# EUROPEAN PATENT SPECIFICATION

(11) **EP 3 191 186 B1**
(45) Date of publication and mention of the grant of the patent: **27.01.2021**
(21) Application number: 15840643.9
(22) Date of filing: 09.09.2015
(51) Int. Cl.: A61P 25/28, A61K 38/43, G01N 33/68, A61K 38/17, A61K 38/44

(54) **COMPOSITIONS AND METHODS OF USING INTERLEUKIN-4 INDUCED GENE 1 (IL4I1)**
ZUSAMMENSETZUNGEN UND VERFAHREN ZUR VERWENDUNG VON INTERLEUKIN-4-INDUZIERTEM GEN 1 (IL4I1)
COMPOSITIONS ET PROCÉDÉS D'UTILISATION DE L'IL4I1 (INTERLEUKINE-4 INDUCED GENE 1)

(30) Priority: 10.09.2014 US 201462048781 P
(43) Date of publication of application: 19.07.2017
(73) Proprietor: Georgetown University, Washington, DC 20057 (US)
(72) Inventor: HUANG, Jeffrey, Bethesda, Maryland 20816 (US)
(74) Representative: Gevers & Orès
(86) International application number: PCT/US2015/049196
(87) International publication number: WO 2016/040488

(56) References cited:
- WO-A1-2015/035261
- WO-A2-02/18574
- WO-A2-2007/047335
- CA-A1- 2 899 961
- US-A- 5 695 751
- US-A1- 2003 059 437
- US-A1- 2014 030 734
- BUTOVSKY O ET AL: "Microglia activated by IL-4 or IFN-gamma differentially induce neurogenesis and oligodendrogenesis from adult stem/progenitor cells", MOLECULAR AND CELLULAR NEUROSCIENCES, SAN DIEGO, US, vol. 31, no. 1, 1 January 2006 (2006-01-01), pages 149-160, XP024908096, ISSN: 1044-7431, DOI: 10.1016/J.MCN.2005.10.006 [retrieved on 2006-01-01]
- D. A. YOUNG ET AL: "IL-4, IL-10, IL-13, and TGF- from an Altered Peptide Ligand-Specific Th2 Cell Clone Down-Regulate Adoptive Transfer of Experimental Autoimmune Encephalomyelitis", THE JOURNAL OF IMMUNOLOGY, vol. 164, no. 7, 1 April 2000 (2000-04-01) , pages 3563-3572, XP055454280, US ISSN: 0022-1767, DOI: 10.4049/jimmunol.164.7.3563
- PUIFFE, ML ET AL.: 'Antibacterial Properties Of the Mammalian L-Amino Acid Oxidase IL 4I1.' PLOS ONE. vol. 8, no. 1, 23 January 2013, page 5, XP055384626
- ZHANG, H ET AL.: 'Central Nervous System Remyelination In Culture - A Tool For Multiple Sclerosis Research.' EXP NEUROL. vol. 230, no. 1, July 2011, pages 138 - 148, XP055261770

### SEQUENCE LISTING

<110> GEORGETOWN UNIVERSITY HUANG, Jeffrey
<120> COMPOSITIONS AND METHODS OF USING INTERLEUKIN-4 INDUCED GENE 1 (IL4i1)
<130> 036681-5035-WO
<150> US 62/048,781
   <151> 2014-09-10
<160> 4
<170> PatentIn version 3.5
<210> 1
   <211> 567
   <212> PRT
   <213> Homo sapiens
<400> 1
   Met Ala Pro Leu Ala Leu His Leu Leu Val Leu Val Pro Ile Leu Leu
   1               5                   10                  15
   Ser Leu Val Ala Ser Gln Asp Trp Lys Ala Glu Arg Ser Gln Asp Pro
               20                  25                  30
   Phe Glu Lys Cys Met Gln Asp Pro Asp Tyr Glu Gln Leu Leu Lys Val
           35                  40                  45
   Val Thr Trp Gly Leu Asn Arg Thr Leu Lys Pro Gln Arg Val Ile Val
       50                  55                  60
   Val Gly Ala Gly Val Ala Gly Leu Val Ala Ala Lys Val Leu Ser Asp
   65                  70                  75                  80
   Ala Gly His Lys Val Thr Ile Leu Glu Ala Asp Asn Arg Ile Gly Gly
                   85                  90                  95
   Arg Ile Phe Thr Tyr Arg Asp Gln Asn Thr Gly Trp Ile Gly Glu Leu
               100                 105                 110
   Gly Ala Met Arg Met Pro Ser Ser His Arg Ile Leu His Lys Leu Cys
           115                 120                 125
   Gln Gly Leu Gly Leu Asn Leu Thr Lys Phe Thr Gln Tyr Asp Lys Asn
       130                 135                 140
   Thr Trp Thr Glu Val His Glu Val Lys Leu Arg Asn Tyr Val Val Glu
   145                 150                 155                 160
   Lys Val Pro Glu Lys Leu Gly Tyr Ala Leu Arg Pro Gln Glu Lys Gly
   165 170 175
   His Ser Pro Glu Asp Ile Tyr Gln Met Ala Leu Asn Gln Ala Leu Lys
   180 185 190
   Asp Leu Lys Ala Leu Gly Cys Arg Lys Ala Met Lys Lys Phe Glu Arg
   195 200 205
   His Thr Leu Leu Glu Tyr Leu Leu Gly Glu Gly Asn Leu Ser Arg Pro
   210 215 220
   Ala Val Gln Leu Leu Gly Asp Val Met Ser Glu Asp Gly Phe Phe Tyr
   225 230 235 240
   Leu Ser Phe Ala Glu Ala Leu Arg Ala His Ser Cys Leu Ser Asp Arg
   245 250 255
   Leu Gln Tyr Ser Arg Ile Val Gly Gly Trp Asp Leu Leu Pro Arg Ala
   260 265 270
   Leu Leu Ser Ser Leu Ser Gly Leu Val Leu Leu Asn Ala Pro Val Val
   275 280 285
   Ala Met Thr Gln Gly Pro His Asp Val His Val Gln Ile Glu Thr Ser
   290 295 300
   Pro Pro Ala Arg Asn Leu Lys Val Leu Lys Ala Asp Val Val Leu Leu
   305 310 315 320
   Thr Ala Ser Gly Pro Ala Val Lys Arg Ile Thr Phe Ser Pro Pro Leu
   325 330 335
   Pro Arg His Met Gln Glu Ala Leu Arg Arg Leu His Tyr Val Pro Ala
   340 345 350
   Thr Lys Val Phe Leu Ser Phe Arg Arg Pro Phe Trp Arg Glu Glu His
   355 360 365
   Ile Glu Gly Gly His Ser Asn Thr Asp Arg Pro Ser Arg Met Ile Phe
   370 375 380
   Tyr Pro Pro Pro Arg Glu Gly Ala Leu Leu Leu Ala Ser Tyr Thr Trp
   385 390 395 400
   Ser Asp Ala Ala Ala Ala Phe Ala Gly Leu Ser Arg Glu Glu Ala Leu
   405 410 415
   Arg Leu Ala Leu Asp Asp Val Ala Ala Leu His Gly Pro Val Val Arg
   420 425 430
   Gln Leu Trp Asp Gly Thr Gly Val Val Lys Arg Trp Ala Glu Asp Gln
   435 440 445
   His Ser Gln Gly Gly Phe Val Val Gln Pro Pro Ala Leu Trp Gln Thr
   450 455 460
   Glu Lys Asp Asp Trp Thr Val Pro Tyr Gly Arg Ile Tyr Phe Ala Gly
   465 470 475 480
   Glu His Thr Ala Tyr Pro His Gly Trp Val Glu Thr Ala Val Lys Ser
   485 490 495
   Ala Leu Arg Ala Ala Ile Lys Ile Asn Ser Arg Lys Gly Pro Ala Ser
   500 505 510
   Asp Thr Ala Ser Pro Glu Gly His Ala Ser Asp Met Glu Gly Gln Gly
   515 520 525
   His Val His Gly Val Ala Ser Ser Pro Ser His Asp Leu Ala Lys Glu
   530 535 540
   Glu Gly Ser His Pro Pro Val Gln Gly Gln Leu Ser Leu Gln Asn Thr
   545 550 555 560
   Thr His Thr Arg Thr Ser His
   565
<210> 2
   <211> 546
   <212> PRT
   <213> Homo sapiens
<400> 2
   Gln Asp Trp Lys Ala Glu Arg Ser Gln Asp Pro Phe Glu Lys Cys Met
   1 5 10 15
   Gln Asp Pro Asp Tyr Glu Gln Leu Leu Lys Val Val Thr Trp Gly Leu
   20 25 30
   Asn Arg Thr Leu Lys Pro Gln Arg Val Ile Val Val Gly Ala Gly Val
   35 40 45
   Ala Gly Leu Val Ala Ala Lys Val Leu Ser Asp Ala Gly His Lys Val
   50 55 60
   Thr Ile Leu Glu Ala Asp Asn Arg Ile Gly Gly Arg Ile Phe Thr Tyr
   65                  70                  75                  80
   Arg Asp Gln Asn Thr Gly Trp Ile Gly Glu Leu Gly Ala Met Arg Met
                   85                  90                  95
   Pro Ser Ser His Arg Ile Leu His Lys Leu Cys Gln Gly Leu Gly Leu
               100                 105                 110
   Asn Leu Thr Lys Phe Thr Gln Tyr Asp Lys Asn Thr Trp Thr Glu Val
           115                 120                 125
   His Glu Val Lys Leu Arg Asn Tyr Val Val Glu Lys Val Pro Glu Lys
       130                 135                 140
   Leu Gly Tyr Ala Leu Arg Pro Gln Glu Lys Gly His Ser Pro Glu Asp
   145                 150                 155                 160
   Ile Tyr Gln Met Ala Leu Asn Gln Ala Leu Lys Asp Leu Lys Ala Leu
                   165                 170                 175
   Gly Cys Arg Lys Ala Met Lys Lys Phe Glu Arg His Thr Leu Leu Glu
               180                 185                 190
   Tyr Leu Leu Gly Glu Gly Asn Leu Ser Arg Pro Ala Val Gln Leu Leu
           195                 200                 205
   Gly Asp Val Met Ser Glu Asp Gly Phe Phe Tyr Leu Ser Phe Ala Glu
       210                 215                 220
   Ala Leu Arg Ala His Ser Cys Leu Ser Asp Arg Leu Gln Tyr Ser Arg
   225                 230                 235                 240
   Ile Val Gly Gly Trp Asp Leu Leu Pro Arg Ala Leu Leu Ser Ser Leu
                   245                 250                 255
   Ser Gly Leu Val Leu Leu Asn Ala Pro Val Val Ala Met Thr Gln Gly
               260                 265                 270
   Pro His Asp Val His Val Gln Ile Glu Thr Ser Pro Pro Ala Arg Asn
           275                 280                 285
   Leu Lys Val Leu Lys Ala Asp Val Val Leu Leu Thr Ala Ser Gly Pro
       290                 295                 300
   Ala Val Lys Arg Ile Thr Phe Ser Pro Pro Leu Pro Arg His Met Gln
   305 310 315 320
   Glu Ala Leu Arg Arg Leu His Tyr Val Pro Ala Thr Lys Val Phe Leu
   325 330 335
   Ser Phe Arg Arg Pro Phe Trp Arg Glu Glu His Ile Glu Gly Gly His
   340 345 350
   Ser Asn Thr Asp Arg Pro Ser Arg Met Ile Phe Tyr Pro Pro Pro Arg
   355 360 365
   Glu Gly Ala Leu Leu Leu Ala Ser Tyr Thr Trp Ser Asp Ala Ala Ala
   370 375 380
   Ala Phe Ala Gly Leu Ser Arg Glu Glu Ala Leu Arg Leu Ala Leu Asp
   385 390 395 400
   Asp Val Ala Ala Leu His Gly Pro Val Val Arg Gln Leu Trp Asp Gly
   405 410 415
   Thr Gly Val Val Lys Arg Trp Ala Glu Asp Gln His Ser Gln Gly Gly
   420 425 430
   Phe Val Val Gln Pro Pro Ala Leu Trp Gln Thr Glu Lys Asp Asp Trp
   435 440 445
   Thr Val Pro Tyr Gly Arg Ile Tyr Phe Ala Gly Glu His Thr Ala Tyr
   450 455 460
   Pro His Gly Trp Val Glu Thr Ala Val Lys Ser Ala Leu Arg Ala Ala
   465 470 475 480
   Ile Lys Ile Asn Ser Arg Lys Gly Pro Ala Ser Asp Thr Ala Ser Pro
   485 490 495
   Glu Gly His Ala Ser Asp Met Glu Gly Gln Gly His Val His Gly Val
   500 505 510
   Ala Ser Ser Pro Ser His Asp Leu Ala Lys Glu Glu Gly Ser His Pro
   515 520 525
   Pro Val Gln Gly Gln Leu Ser Leu Gln Asn Thr Thr His Thr Arg Thr
   530 535 540
   Ser His
   545
<210> 3
   <211> 36
   <212> PRT
   <213> Human immunodeficiency virus type 1
<400> 3
   Cys Phe Ile Thr Lys Ala Leu Gly Ile Ser Tyr Gly Arg Lys Lys Arg
   1               5                   10                  15
   Arg Gln Arg Arg Arg Pro Pro Gln Gly Ser Gln Thr His Gln Val Ser
               20                  25                  30
   Leu Ser Lys Gln
           35
<210> 4
   <211> 10
   <212> PRT
   <213> Human immunodeficiency virus type 1
<400> 4
   Gly Arg Lys Lys Arg Arg Gln Arg Arg Arg
   1               5                   10

## Description

### Background of the Invention

### Field of the Invention

The description is directed to methods of promoting myelin formation in central nervous system (CNS) tissue in a subject in need thereof, the method comprising administering to the subject a therapeutically effective amount of interleukin-4 induced gene 1 (IL4-il) protein. The description is also directed to methods of monitoring the progression of conditions marked by an impairment of myelin formation in the CNS comprising assessing the levels or activity of IL4-i1 in activated macrophages obtained from the subject.

### Background of the Invention

Multiple sclerosis (MS) is a progressively disabling, chronic inflammatory neurological disorder that affects 12.5 million adults worldwide. It is characterized by sustained axonal dysfunction and chronic neurodegeneration due to persistent inflammatory destruction of oligodendrocyte-derived myelin in the brain, optic nerve and spinal cord. In the early phase of MS, efficient myelin regeneration (remyelination) occurs following demyelination, where neighboring adult oligodendrocyte precursor cells (OPC) are activated and migrate to lesions and differentiate into mature, remyelinating oligodendrocytes (OL). This spontaneous regenerative process is crucial for CNS repair and limits the axonal dysfunction and neurodegeneration that otherwise occurs with myelin loss. However, the ability to regenerate myelin becomes limited with disease progression, such that the increased number of unhealed, denuded axons in the CNS leads to impaired axonal conduction and axoplasmic transport, insufficient trophic/metabolic support by the surrounding oligodendrocytes/myelin, and increased oxidative stress, resulting in chronic neurodegeneration that manifests as the accumulation of irreversible clinical disability in patients.

Studies in chronic MS lesions indicate that OPCs are able to migrate to demyelinated lesions but fail to differentiate into remyelinating OL, suggesting that signals required to promote OL differentiation are absent or suppressed. Indeed, previous pioneering studies in rodents that recapitulated the chronic demyelination environment have demonstrated that OPCs recruited to lesions remain competent to myelinate, but are unable to do so unless an acute inflammatory stimulus were delivered. Moreover, altered inflammatory activity has been observed in chronic MS lesions displaying widespread demyelination and neurodegeneration. These studies suggest that certain inflammation-derived, pro-regenerative factors may be needed in demyelinated lesions to stimulate OL differentiation from OPCs.

Macrophages are known to play a critical role in remyelination, ranging from debris clearance to promoting oligodendrocyte (OL) differentiation. Activated microglia/macrophages, which constitute a major component of innate immunity in the brain, are involved in a spectrum of activities after demyelination, ranging from debris clearance to myelin repair. Macrophages can be broadly categorized as (1) classically-activated "M1" macrophages, which exhibit cytotoxic properties, and (2) alternatively-activated "M2" macrophages, which exhibit anti-inflammatory and tissue remodeling properties.

It appears that M2 macrophages in CNS are required for remyelination, thus providing an important clue to how the innate immune response promotes myelin repair. Macrophages may thus be excellent target for pro-remyelinating therapy. The mechanisms of how macrophages regulate OL differentiation/remyelination, however, remain poorly understood.

IL4i1 is a secreted L-amino acid oxidase (LAAO) expressed by macrophages and dendritic cells, and displays antibiotic and immunosuppressive properties. Moreover, the IL4i1 gene has been mapped to a chromosomal region that is a hotspot for autoimmune disease susceptibility, including MS. But the role of the gene's expression in MS has not been fully described. IL-4 downregulates adoptive transfer of experimental autoimmune encephalomyelitis (EAE) (Young et al., J. Immunol., 164:3563-3572, 2000). Exactly how IL4i1 regulates inflammation and whether IL4i1 is associated with M2 macrophage activity thus remains unknown.

The identification of critical pro-remyelinating signals would aide in the development of regenerative therapeutics to enhance remyelination in MS and potentially prevent disease progression.

### Summary of the Invention

The subject matter of the invention is defined by claims 1 to 18.

The invention is directed to a composition for use in the treatment of multiple sclerosis or other condition marked by an impairment of myelin regeneration in the CNS in a subject, said composition comprising IL4-i1 protein.

The invention is also directed to a method of diagnosing a subject as having a predisposition to having a condition marked by impairment of myelin regeneration in the CNS, the method comprising determining activity or levels of IL4-i1 protein in a sample from the subject, wherein a reduction in the activity or levels of IL4-i1 in a sample from the subject is indicative that the subject has a predisposition to having a condition marked by demyelination or an impairment of myelin regeneration.

The invention is also directed to a method of monitoring the progression in a subject of a condition marked by impairment of myelin regeneration in CNS tissue and/or axonal loss, the method comprising determining for at least two time points activity levels of IL4-i1 protein in a sample from the subject, wherein a reduction in the activity or levels of IL4-i1 in a sample from the subject over time is indicative that the condition marked by an impairment of myelin regeneration is progressing in the subject and wherein an increase in the activity or levels of IL4-i1 in the subject over time is indicative that the condition marked by an impairment of myelin regeneration is regressing in the subject.

The description is directed to methods of promoting myelin formation in central nervous system (CNS) tissue in a subject in need thereof, the method comprising administering to the subject a therapeutically effective amount of interleukin-4 induced gene 1 (IL4-i1) protein.

The description is also directed to methods of monitoring the progression of conditions marked by an impairment of myelin formation in the CNS comprising assessing the levels or activity of IL4-i1 in activated macrophages obtained from the subject.

### Brief Description of the Drawings

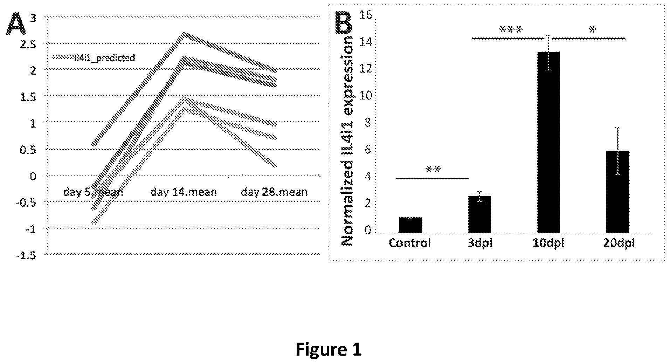

FIGURE 1 depicts IL4i1 expression during remyelination. (A) The data shows several genes displaying a transient increase in expression at 14 dpl. X-axis shows 3 timepoints postlesion. The Y-axis shows gene expression relative to overall signal of all genes detected at each time point (from remyelination transcriptome. (B) qPCR validates IL4i1 expression in lysolecithin-demyelinated mouse spinal cord.

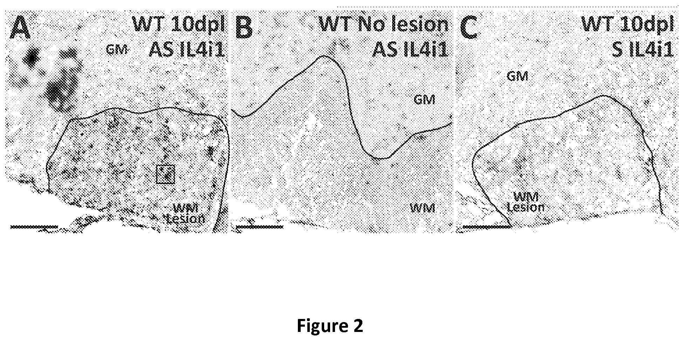

FIGURE 2 depicts *in situ* hybridization of IL4i1 in focally demyelinated mouse spinal cord white matter at 15 dpl. (A) Antisense IL4i1 RNA labels spinal cord white matter lesion (WM, lesion encircled). Inset shows labeling of cells with rounded appearance. (B) In unlesioned tissue, IL4i1 is barely detected in WM but is detected in randomly distributed cells in the grey matter (GM).(C) Sense control does not label WM lesion.

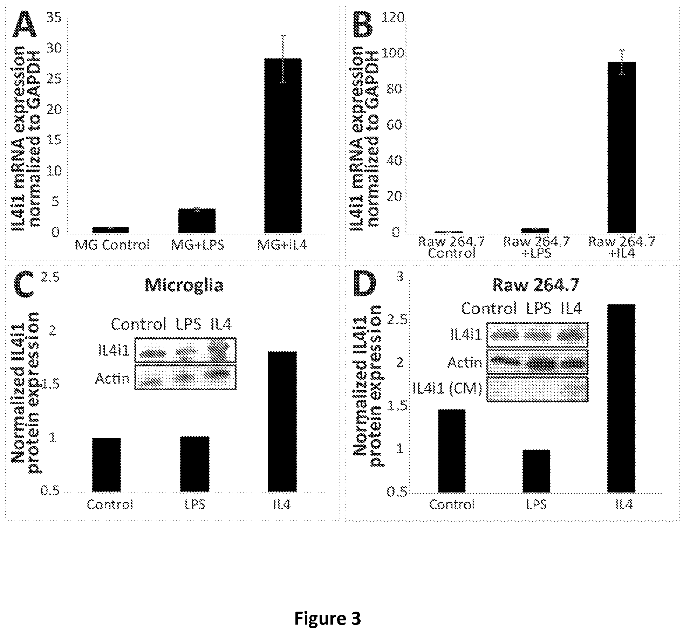

FIGURE 3 depicts real time quantitative PCR analysis of (A) microglia (MG) and (B) RAW 264.7 cells treated with vehicle (control), LPS or IL4. IL4i1 expression is upregulated following IL4 induction of MG and RAW 264.7 cells into M2 macrophages. Western blot analysis confirmed that IL4i1 protein is upregulated following IL4 addition to (C) MG and (D) RAW 264.7 cells.

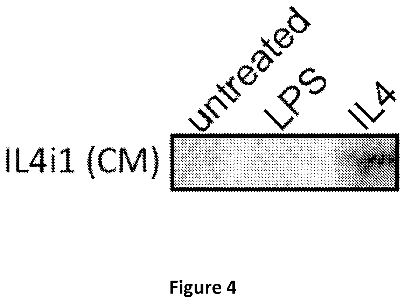

FIGURE 4 depicts a western blot of conditioned media (CM) from untreated, LPS treated or IL4 treated RAW 264.7 cells. IL4i1 is detected only in IL4 treated cells, suggesting that macrophage cells secrete IL4i1 when induced into the M2 phenotype.

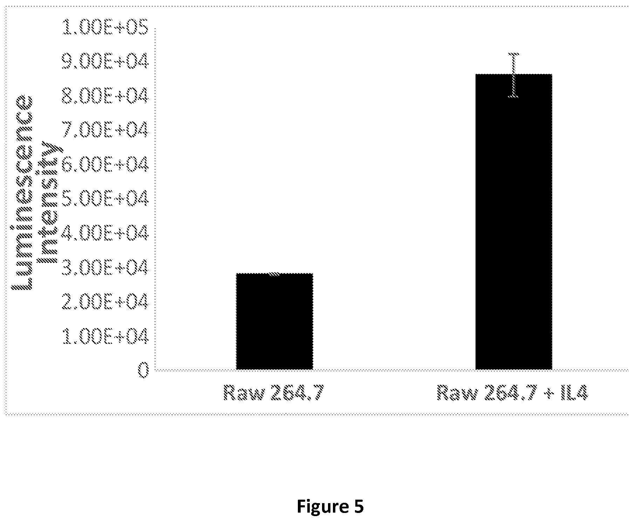

FIGURE 5 depicts a hydrogen peroxide (H₂O₂) detection assay on RAW 264.7 cells and RAW 264.7 cells treated with IL4. The graph shows that H₂O₂ level increases upon IL4 induction.

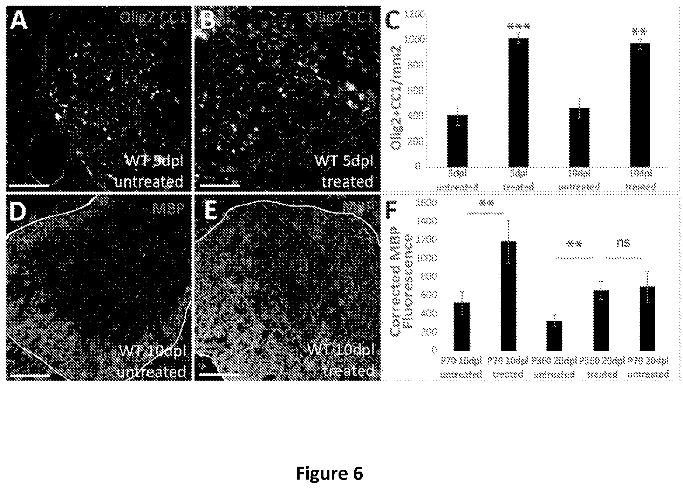

FIGURE 6 depicts immunofluorescence analysis of oligodendrocyte lineage cells and remyelination in postnatal day 70 (P70) mice that received a mouse recombinant IL4i1 (rmIL4i1) protein in focally demyelinated spinal cord. (A and B) Treatment with rmIL4i1 or vehicle resulted in significantly more Olig2⁺/CC1⁺ oligodendrocytes in lesions at 5 and 10dpl. (C) Quantification of Olig2⁺/CC1⁺ cells. (D and E) rmIL4i1 treatment compared to untreated resulted in more myelin basic protein (MBP) labeling in the lesion (white outline) at 10dpl. (F) Quantification of MBP labeling in P70 mice. Increased MBP labeling in lesions was also detected in postnatal day 360 (P360) mice that received rmIL4i1, which indicates rmIL4i1 was able to correct or improve the remyelination inefficiency that normally occurs in geriatric mice.

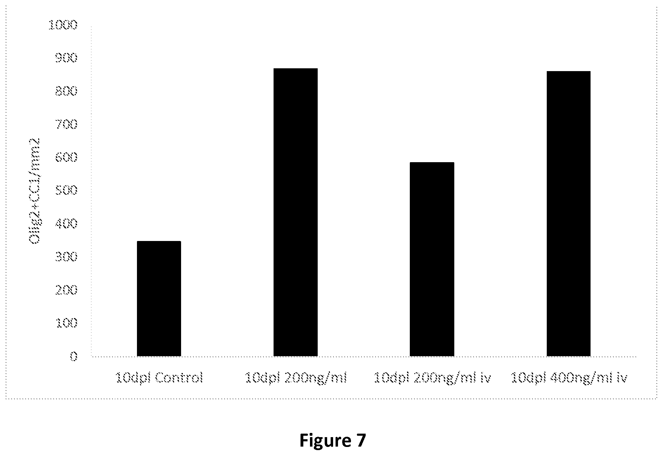

FIGURE 7 depicts intravenous injection of recombinant IL4i1 promoting CNS remyelination. The graph shows intravenous delivery of IL4i1 (200 ng/ml or 400 ng/ml) significantly increased the number of mature oligodendrocytes (CC1+Olig2+) in spinal cord lesions at 10 days post lesion, compared to untreated spinal cord. Injection of 400 ng/ml IL4i1 intravenously was as effective as injection of 200 ng/ml IL4i1 by focal injection into the lesion.

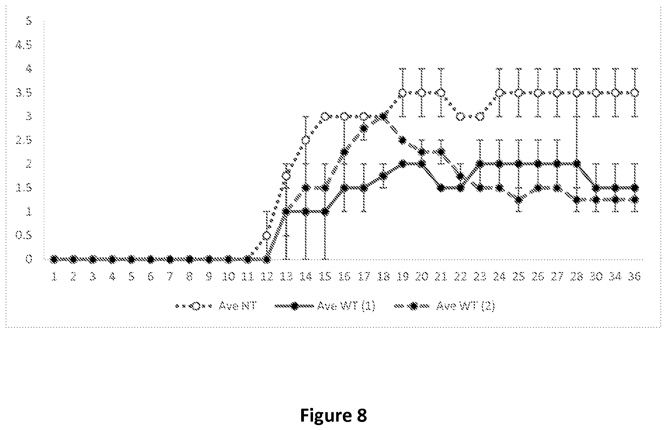

FIGURE 8 depicts intravenous injection of recombinant IL4i1 significantly improving clinical symptoms in mice with experimental autoimmune encephalitis (EAE), an immune-mediated demyelination model of multiple sclerosis. The graph shows clinical scores on the y-axis and days post EAE induction on the x-axis. Mice were given recombinant IL4i1 at 13 days post EAE induction when symptoms began (clinical score 1.5; limp tail). Treated mice displayed less severe symptoms over time compared to untreated mice that displayed worsening symptoms, such as hindlimb unsteady/paralysis (clinical score 3-3.5). Compared to untreated mice (NT; open circles), mice (WT1; closed circles, solid line) given a single dose (400 ng/ml) of recombinant IL4i1 did not display worsening symptoms and remained relatively stable (score 2) for at least 40 days. When mice (WT2; closed circles, dashed line) were given a high dose of recombinant IL4i1 (700 ng/ml) at 17 days post induction during the peak of clinical symptoms (clinical score 3), they recovered from unsteady hindlimb movement and showed visible improvement in movement over time and remained stable (clinical score 1.5) until at least 40 days post induction. This experiment indicates the peripheral delivery (such as through intravenous injection) of recombinant IL4i1 improves clinical symptoms in immune-mediated demyelination.

### Detailed Description of the Invention

In an aspect, the invention relates to a composition for use in the treatment of multiple sclerosis or other condition marked by an impairment of myelin regeneration in the CNS in a subject, said composition comprising IL4-i1 protein.

In an embodiment, the invention relates to the composition for its use as defined above, wherein the subject has been diagnosed with multiple sclerosis (MS), Parkinson's Disease (PD), or has received a traumatic brain injury or a spinal cord injury prior to the administration of the IL4-i1 protein.

In an embodiment, the invention relates to the composition for its use as defined above, wherein the subject has a neuro-inflammatory disorder causing impairment of myelin regeneration and/or axonal loss prior to the administration of the IL4-i1 protein.

In an embodiment, the invention relates to the composition for its use as defined above, wherein the neuro-inflammatory disorder causes demyelination.

In an embodiment, the invention relates to the composition for its use as defined above, wherein the IL4-i1 protein is administered directly to the site in the CNS that is in need of myelin formation.

In an embodiment, the invention relates to the composition for its use as defined above, wherein the IL4-i1 protein is administered systemically and/or peripherally to the subject.

In an aspect, the invention relates to a method of diagnosing a subject as having a predisposition to having a condition marked by impairment of myelin regeneration in the CNS, the method comprising determining activity or levels of IL4-i1 protein in a sample from the subject, wherein a reduction in the activity or levels of IL4-i1 in a sample from the subject is indicative that the subject has a predisposition to having a condition marked by demyelination or an impairment of myelin regeneration.

In an embodiment, the invention relates to the method as defined above, wherein the activity or levels of IL4-i1 are determined in activated macrophages.

In an embodiment, the invention relates to the method as defined above, wherein the activated macrophages are M2 activated macrophages.

In an embodiment, the invention relates to the method as defined above, wherein the condition marked by an impairment of myelin regeneration in the CNS is multiple sclerosis.

In an embodiment, the invention relates to the method as defined above, wherein the condition marked by an impairment of myelin regeneration in the CNS is traumatic brain injury or spinal cord injury.

In an embodiment, the invention relates to the method as defined above, wherein the condition marked by impairment of myelin is demyelination.

In an aspect, the invention relates to a method of monitoring the progression in a subject of a condition marked by impairment of myelin regeneration in CNS tissue and/or axonal loss, the method comprising determining for at least two time points activity levels of IL4-i1 protein in a sample from the subject, wherein a reduction in the activity or levels of IL4-i1 in a sample from the subject over time is indicative that the condition marked by an impairment of myelin regeneration is progressing in the subject and wherein an increase in the activity or levels of IL4-i1 in the subject over time is indicative that the condition marked by an impairment of myelin regeneration is regressing in the subject.

In an embodiment, the invention relates to the method as defined above, wherein the subject is receiving a treatment for the condition marked by an impairment of myelin regeneration and wherein a reduction in the activity or levels of IL4-i1 in a sample from the subject over time is indicative that the treatment is not effective in slowing the progression of the condition marked by an impairment of myelin regeneration in the subject.

In an embodiment, the invention relates to the method as defined above, wherein the subject is receiving a treatment for the condition marked by an impairment of myelin regeneration and wherein an increase in the activity or levels of IL4-i1 in a sample from the subject over time is indicative that the treatment is effective in slowing the progression of the condition marked by an impairment of myelin regeneration in the subject.

In an embodiment, the invention relates to the method as defined avove, wherein the condition marked by an impairment of myelin is demyelination.

In an embodiment, the invention relates to the method as defined above, wherein the subject is not receiving treatment for the condition marked by an impairment of myelin regeneration prior to the determining the activity or levels of IL4-i1.

In an embodiment, the invention relates to the method as defined above, wherein the activity or levels of IL4-i1 are determined in activated macrophages.

The description is directed to methods of promoting myelin formation in central nervous system (CNS) tissue in a subject in need thereof, the method comprising administering to the subject a therapeutically effective amount of interleukin-4 induced gene 1 (IL4-il) protein.

Myelin is a well-known type of cell membrane that surrounds axons and other extensions of nerve cells in the central and peripheral nervous systems. Myelin is actually a lipoprotein membrane comprising a biomolecular lipid layer that lies between the monomolecular layers of protein and is spirally wrapped around the internodal segment of a nerve fiber. The principal functions of myelin include, but are not limited to, metabolic isolation and acceleration of nerve impulse conduction, along with supporting and barrier functions.

As used herein, the phrases "promoting myelination" or "promoting myelin formation" is used to mean myelin formation occurs quicker or more easily in cells, tissues, organs or subjects when treated with the peptides of the present invention compared to untreated ("control") cells, tissues, organs or subjects. Methods for determining the rate of myelin formation are well-known in the art and include such assays as immunofluorescence analysis to label oligodendrocyte precursors, differentiated oligodendrocytes and myelin; morphological analysis under light microscope of semi-thin resin sections of lesioned CNS tissues; ultrastructural analysis by electron microscopy. Remyelination is readily distinguished from normally myelinated axons because myelin sheaths from remyelination are relatively thinner. For example, fluorescent microscopy methods of assessing myelin formation are disclosed in Bilderback, T., et al., J. Neurosci. Res., 49(4):497-507 (1997).

For the purposes of the present invention, determining the promotion, or lack thereof, of myelin formation can be performed on cells of similar disease or metabolic state, or even on cells of different disease or metabolic state. For example, when assessing if the treatment methods result in promotion of myelin formation, the treatment methods may be applied to cells, tissues, organs or subjects in which myelin formation is known to be impaired, *e*.*g*., cells from multiple sclerosis (MS) subjects, and the untreated cells, tissues organs or subjects may be from cells, tissues, organs or subjects in which myelin formation is known to function normally or is also known to be impaired. Similarly, when assessing if the treatment methods result in promotion of myelin formation, the treatment methods may be applied to cells, tissues, organs or subjects in which myelin formation is known to be functioning normally, and the untreated cells, tissues organs or subjects may be from cells, tissues, organs or subjects in which myelin formation is known function normally or is known to be impaired. Thus, the term "control" or "control group" when used in conjunction in determining promotion of myelin formation, is not necessarily synonymous with "normal" or "normal group."

The promotion of myelin formation may, but need not, be statistically significant over the untreated group. As long as there is a detectable increase in the ability of cells, tissue, organs or subjects to form myelin over untreated cells, tissue, organs or subjects, then the treatment methods can be said to myelin formation.

In one embodiment the methods are performed on subjects that have been diagnosed with a condition marked by an impairment of myelin formation. As used herein, a condition that is marked by an impairment of myelin formation is used to indicate that queried cells, tissue, organs or subjects have a reduced capacity or rate of myelin formation over rates or capacity that is viewed or accepted as being within the normal limits of myelin formation.

As is commonly understood in the art, diseases of myelin formation can be categorized into two broad classes of diseases: demyelination diseases and dysmyelination diseases. Demyelination conditions are generally marked by insults or injuries to previously normal myelin or normal levels of myelin. Dysmyelination, on the other hand, is marked by an abnormal ability to properly form myelin or by abnormal kinetics associated with normal myelin turnover. The phrase "impaired myelin formation" and the like encompasses both broad categories of diseases involving myelin formation, re-formation or maintenance such that there are generally reduced levels of myelin in cells, tissues, organs and/or subjects when compared to normal levels. In one specific embodiment, the phrase "impaired myelin formation" means a condition marked by demyelination. In another specific embodiment, the phrase "impaired myelin formation" means a condition marked by dysmyelination.

Methods are well known to assess if demyelination and/or dysmyelination are occurring in a particular cell, tissue, organ or subject. For example, Lucchinetti C.F., et al., Brain Pathol., 6(3):259-74 (1996), Chang A., et al., N. Engl. J. Med., 346(3):165-73 (2002), and Polman C.H., et al., Ann. Neurol. 58(6):840-6 (2005), disclose methods of assessing demyelination and/or dysmyelination.

In select embodiments, the methods of the present invention are performed on subjects that have been diagnosed with a condition marked by demyelination and/or dysmyelination. Conditions marked by demyelination and/or dysmyelination include but are not limited to multiple sclerosis (MS), Parkinson's Disease (PD) and traumatic brain injury (TBI)), spinal cord injury (SCI), Alzheimer's Disease (AD), cerebral palsy, periventricular leukomalacia (PVL), schizophrenia, leukoencephalopathies, leukodystrophies, and any form of idiopathic inflammatory demyelinating disease.

To establish the "normal levels" of myelin formation, cells, tissues, organs from individuals or individuals themselves with no apparent symptoms of impairment of myelin formation may be first assessed for their ability to form myelin. Once established, these levels can then be set or determined to establish "normal levels of myelin formation" or "normal ability to form myelin." In one embodiment, normal levels can be ascertained from the same subject when the subject is deemed to possess normal abilities and no signs (clinical or otherwise) of impairment of myelin formation. In one embodiment, "normal levels" are assessed in the same subject from whom the sample is taken prior to the onset of measureable, perceivable or diagnosed impairment of myelin formation. That is, the term "normal" with respect to an ability to form myelin can be used to mean a subject's baseline levels or activity prior to the onset of impairment of myelin formation. These activity levels of myelin formation can then be reassessed periodically and compared to the subject's baseline activity levels. Thus, the present invention also include methods of monitoring the progression of conditions marked by an impairment of myelin formation in a subject, with the methods comprising determining the subject's activity or levels of myelin formation more than once over a period of time. For example, some embodiments of the methods of the present invention will comprise determining the subject's activity levels two, three, four, five, six, seven, eight, nine, 10 or even more times over a period of time, such as a year, two years, three, years, four years, five years, six years, seven years, eight years, nine years or even 10 years or longer. The methods of monitoring a subject's risk of acquiring a condition marked by an impairment of myelin formation would also include embodiments in which the subject's levels are assessed during and after treatment of memory impairment. In other words, the present invention also includes methods of monitoring the efficacy of treatment of a condition marked by impairment of myelin formation by assessing the subject's activity or levels of myelin formation over the course of the treatment and after the treatment.

In another embodiment, "normal levels" or "normal activities" are assessed in a sample from different cells, tissues, organs or individuals from the test or treatment cells, tissues, organs or individuals is assessed. In still another embodiment, the "normal levels" or "normal activities" are assessed in a population of healthy cells, tissues, organs or individuals, the constituents of which display no signed impairment of myelin formation. Thus, the test levels or activity of myelin formation can be compared to normal levels or activities generated from a single normal sample or from more than one normal sample.

As used herein, "contacting," when used in connection with the methods of the present description means bringing the IL4i1 peptides or variants thereof, in proximity to the target cells such that a specific binding event or a biological effect is possible. Thus, contacting can include adding the IL4i1 peptides or variants thereof in culture medium and applying the culture medium to cells in culture. Contacting also encompasses transfecting a cell with at least one vector described herein and allowing the cell to produce the IL4i1 peptides or variants thereof. Of course, contacting would also include administration of the IL4i1 peptides or variants thereof, or pharmaceutical compositions thereof, of the present invention to cells in an intact organism. Compositions for administering the IL4i1 peptides or variants thereof of the present invention have been described herein. Of course, measurements of the levels or activities can fall within a range of values, and values that do not fall within this "normal range" are said to be outside the normal range. These measurements may or may not be converted to a value, number, factor or score as compared to measurements in the "normal range."

As used herein, "administering," and "administer" are used to mean introducing at least one compound comprising at least one of the IL4i1 peptides or variants thereof into a subject. When administration is for the purpose of treatment, the substance is provided at, or after the onset of, a symptom or condition in need of treatment, such as the impairment of myelin formation. The therapeutic administration of this substance serves to attenuate any symptom, or prevent additional symptoms from arising. When administration is for the purposes of preventing a condition from arising ("prophylactic administration"), the IL4i1 peptides or variants thereof are provided in advance of any visible or detectable symptom. The prophylactic administration of the IL4i1 peptides or variants thereof serves to attenuate subsequently arising symptoms or prevent symptoms from arising altogether. The route of administration of the compound includes, but is not limited to, topical, transdermal, intranasal, vaginal, rectal, oral, subcutaneous, intravenous, intraarterial, intramuscular, intraosseous, intraperitoneal, epidural and intrathecal as disclosed herein.

Furthermore, the methods of the description would also include coadministering one or more substances in addition to the IL4i1 peptides or variants thereof. The term "coadminister" indicates that each of at least two compounds is administered during a time frame wherein the respective periods of biological activity or effects overlap. Thus the term includes sequential as well as coextensive administration of the any of the compounds of the present invention. And similar to administering compounds, coadministration of more than one substance can be for therapeutic and/or prophylactic purposes. If more than one substance is coadministered, the routes of administration of the two or more substances need not be the same.

The treatment methods relate to the administration of isolated polypeptides. The terms "peptide," "polypeptide" and "protein" are used interchangeably herein. As used herein, an "isolated polypeptide" is intended to mean a polypeptide that has been completely or partially removed from its native environment. For example, polypeptides that have been removed or purified from cells are considered isolated. In addition, recombinantly produced polypeptides molecules contained in host cells are considered isolated for the purposes of the present invention. Moreover, a peptide that is found in a cell, tissue or matrix in which it is not normally expressed or found is also considered as "isolated" for the purposes of the present invention. Similarly, polypeptides that have been synthesized are considered to be isolated polypeptides. "Purified," on the other hand is well understood in the art and generally means that the peptides are substantially free of cellular material, cellular components, chemical precursors or other chemicals beyond, perhaps, buffer or solvent. "Substantially free" is not intended to mean that other components beyond the novel peptides are undetectable. The peptides of the present description may be isolated or purified.

The amino acid sequence of SEQ ID NO:1 represents the full length amino acid sequence of interleukin 4 induced gene 1 (IL4i1). The protein is generally formed as propeptide, which usually contains, from N-terminus to C-terminus, a signal sequence and the "mature" peptide. For IL4i1 below, the signal sequence occurs from amino acid residues 1-21, and the "mature" peptide is from residues 22-567 of SEQ ID NO:1. The amino acid sequence of hIL4i1 below, is also available within the UniProt Consortium Database as UniProt Accession No.Q96RQ9.

The description therefore provides methods of using isolated peptides comprising an amino acid sequence at least 75%, 80%, 85%, 86%, 87%, 88%, 89%, 90%, 91%, 92%, 93%, 94%, 95%, 96%, 97%, 98%, 99% or 100% identical to the amino acid sequence of SEQ ID NO:1. In one specific embodiment, methods comprise the use of a peptide with an amino acid sequence at least 95% identical to the amino acid sequence of SEQ ID NO:1. In further embodiments, methods using the peptides of the present desription comprise an amino acid sequence 100% identical to the amino acid sequence of SEQ ID NO:1.

The amino acid sequence of SEQ ID NO:2, below, represents the mature IL4i1 protein without the signal sequence and corresponds to amino acid residues 22-567 of SEQ ID NO:1. The description therefore provides methods of using peptides comprising an amino acid sequence at least 75%, 80%, 85%, 86%, 87%, 88%, 89%, 90%, 91%, 92%, 93%, 94%, 95%, 96%, 97%, 98%, 99% or 100% identical to the amino acid sequence of SEQ ID NO:2. In one specific embodiment, the methods utilize peptides with an amino acid sequence at least 95% identical to the amino acid sequence of SEQ ID NO:2. In further embodiments, the methods utilize peptides comprising an amino acid sequence 100% identical to the amino acid sequence of SEQ ID NO:2.

A polypeptide having an amino acid sequence at least, for example, 95% "identical" to a reference an amino acid sequence, e.g., SEQ ID NO:2, is understood to mean that the amino acid sequence of the polypeptide is identical to the reference sequence except that the amino acid sequence may include up to five modifications per each 100 amino acids of the reference amino acid sequence. In other words, to obtain a peptide having an amino acid sequence at least 95% identical to a reference amino acid sequence, up to 5% of the amino acid residues of the reference sequence may be deleted or substituted with another amino acid or a number of amino acids up to 5% of the total amino acids in the reference sequence may be inserted into the reference sequence. These modifications of the reference sequence may occur at the N- terminus or C-terminus positions of the reference amino acid sequence or anywhere between those terminal positions, interspersed either individually among amino acids in the reference sequence or in one or more contiguous groups within the reference sequence.

As used herein, "identity" is a measure of the identity of nucleotide sequences or amino acid sequences compared to a reference nucleotide or amino acid sequence. In general, the sequences are aligned so that the highest order match is obtained. "Identity" per se has an art-recognized meaning and can be calculated using published techniques. (*See, e.g.,* Computational Molecular Biology, Lesk, A. M., ed., Oxford University Press, New York (1988); Biocomputing: Informatics And Genome Projects, Smith, D. W., ed., Academic Press, New York (1993); Computer Analysis of Sequence Data, Part I, Griffin, A. M., and Griffin, H. G., eds., Humana Press, New Jersey (1994); von Heinje, G., Sequence Analysis In Molecular Biology, Academic Press (1987); and Sequence Analysis Primer, Gribskov, M. and Devereux, J., eds., M Stockton Press, New York (1991)). While there are several methods to measure identity between two polynucleotide or polypeptide sequences, the term "identity" is well known to skilled artisans (Carillo, H. & Lipton, D., Siam J Applied Math 48:1073 (1988)). Methods commonly employed to determine identity or similarity between two sequences include, but are not limited to, those disclosed in Guide to Huge Computers, Martin J. Bishop, ed., Academic Press, San Diego (1994) and Carillo, H. & Lipton, D., Siam J Applied Math 48:1073 (1988). Computer programs may also contain methods and algorithms that calculate identity and similarity. Examples of computer program methods to determine identity and similarity between two sequences include, but are not limited to, GCG program package (Devereux, J., et al., Nucleic Acids Research 12(i):387 (1984)), BLASTP, ExPASy, BLASTN, FASTA (Atschul, S. F., et al., J Molec Biol 215:403 (1990)) and FASTDB. Examples of methods to determine identity and similarity are discussed in Michaels, G. and Garian, R., Current Protocols in Protein Science, Vol 1, John Wiley & Sons, Inc. (2000).

In one embodiment of the description, the algorithm used to determine identity between two or more polypeptides is BLASTP. In another embodiment of the present description, the algorithm used to determine identity between two or more polypeptides is FASTDB, which is based upon the algorithm of Brutlag et al. (Comp. App. Biosci. 6:237-245 (1990)). In a FASTDB sequence alignment, the query and reference sequences are amino sequences. The result of sequence alignment is in percent identity. In one embodiment, parameters that may be used in a FASTDB alignment of amino acid sequences to calculate percent identity include, but are not limited to: Matrix=PAM, k-tuple=2, Mismatch Penalty=1, Joining Penalty=20, Randomization Group Length=0, Cutoff Score=1, Gap Penalty=5, Gap Size Penalty 0.05, Window Size=500 or the length of the subject amino sequence, whichever is shorter.

If the reference sequence is shorter or longer than the query sequence because of N-terminus or C-terminus additions or deletions, but not because of internal additions or deletions, a manual correction can be made, because the FASTDB program does not account for N-terminus and C-terminus truncations or additions of the reference sequence when calculating percent identity. For query sequences truncated at the N- or C- termini, relative to the reference sequence, the percent identity is corrected by calculating the number of residues of the query sequence that are N- and C- terminus to the reference sequence that are not matched/aligned, as a percent of the total bases of the query sequence. The results of the FASTDB sequence alignment determine matching/alignment. The alignment percentage is then subtracted from the percent identity, calculated by the above FASTDB program using the specified parameters, to arrive at a final percent identity score. This corrected score can be used for the purposes of determining how alignments "correspond" to each other, as well as percentage identity. Residues of the reference sequence that extend past the N- or C-termini of the query sequence may be considered for the purposes of manually adjusting the percent identity score. That is, residues that are not matched/aligned with the N- or C-termini of the comparison sequence may be counted when manually adjusting the percent identity score or alignment numbering.

For example, a 90 amino acid residue query sequence is aligned with a 100 residue reference sequence to determine percent identity. The deletion occurs at the N-terminus of the query sequence and therefore, the FASTDB alignment does not show a match/alignment of the first 10 residues at the N-terminus. The 10 unpaired residues represent 10% of the reference sequence (number of residues at the N- and C-termini not matched/total number of residues in the reference sequence) so 10% is subtracted from the percent identity score calculated by the FASTDB program. If the remaining 90 residues were perfectly matched (100% alignment) the final percent identity would be 90% (100% alignment - 10% unmatched overhang). In another example, a 90 residue query sequence is compared with a 100 reference sequence, except that the deletions are internal deletions. In this case the percent identity calculated by FASTDB is not manually corrected, since there are no residues at the N- or C-termini of the subject sequence that are not matched/aligned with the query. In still another example, a 110 amino acid query sequence is aligned with a 100 residue reference sequence to determine percent identity. The addition in the query occurs at the N-terminus of the query sequence and therefore, the FASTDB alignment may not show a match/alignment of the first 10 residues at the N-terminus. If the remaining 100 amino acid residues of the query sequence have 95% identity to the entire length of the reference sequence, the N-terminal addition of the query would be ignored and the percent identity of the query to the reference sequence would be 95%.

As used herein, the terms "correspond(s) to" and "corresponding to," as they relate to sequence alignment, are intended to mean enumerated positions within the reference protein, e.g., wild-type IL4i1, and those positions in a mutant or related IL4i1 that align with the positions on the reference protein. Thus, when the amino acid sequence of a subject peptide is aligned with the amino acid sequence of a reference IL4i1, e.g., SEQ ID NO:2, the amino acids in the subject sequence that "correspond to" certain enumerated positions of the reference sequence are those that align with these positions of the reference sequence, e.g., SEQ ID NO:2, but are not necessarily in these exact numerical positions of the reference sequence. Methods for aligning sequences for determining corresponding amino acids between sequences are described herein. Accordingly, the description provides novel peptides whose sequences correspond to the sequence of SEQ ID NO:1 or 2.

The description further embraces other species, preferably mammalian, homologs with amino acid sequences that correspond to the IL4i1. Species homologs, sometimes referred to as "orthologs," in general, share at least 35%, 40%, 45%, 50%, 60%, 65%, 70%, 75%, 80%, 85%, 90%, 95%, 96%, 97%, 98%, or 99% sequence identity with the human version of the proteins. Such corresponding sequences account for IL4i1 protein from across a variety of species, such as canine, feline, mouse, rat, rabbit, monkey, etc. of IL4i1.

In another embodiment, the description provides novel peptides whose sequences correspond to the sequence of SEQ ID NO:1 or 2 and retain at least some minimal function.

IL4i1 with an additional methionine residue at position -1 (Met-1-peptide) are contemplated, as are variants with additional methionine and lysine residues at positions -2 and -1 (Met-2-Lys-1-peptide). Variants of the IL4i1 with additional Met, Met-Lys, or Lys residues (or one or more basic residues in general) are particularly useful for enhanced recombinant protein production in bacterial host cells.

Variants resulting from insertion of the polynucleotide encoding the IL4i1 into an expression vector system are also contemplated. For example, variants (usually insertions) may arise from when the amino terminus and/or the carboxy terminus of IL4i1 is/are fused to another polypeptide.

In another aspect, the description provides deletion variants wherein one or more amino acid residues in the IL4i1 protein are removed. Deletions can be effected at one or both termini of the peptide, or with removal of one or more non-terminal amino acid residues of the peptide. Deletion variants, therefore, include all fragments of the IL4i1 peptides disclosed herein.

Within the confines of the disclosed percent identity, the description also relates to substitution variants of the disclosed polypeptides of the description. Substitution variants include those polypeptides wherein one or more amino acid residues of IL4i1 are removed and replaced with alternative residues. In one aspect, the substitutions are conservative in nature; however, the description embraces substitutions that are also non-conservative. Conservative substitutions for this purpose may be defined as set out in the tables below. Amino acids can be classified according to physical properties and contribution to secondary and tertiary protein structure. A conservative substitution is recognized in the art as a substitution of one amino acid for another amino acid that has similar properties. Exemplary conservative substitutions are set out in below.

**Table I - Conservative Substitutions**

| SIDE CHAIN CHARACTERISTIC | AMINO ACID | | | |
|---|---|---|---|---|
| Aliphatic | | | | |
| Non-polar | G | A | P | |
| | I | L | V | |
| Polar - uncharged | C | S | T | M |
| | N | Q | | |
| Polar - charged | D | E | | |
| | K | R | | |
| Aromatic | H | F | W | Y |
| Other | N | Q | D | E |

Alternatively, conservative amino acids can be grouped as described in Lehninger, [Biochemsitry, Second Edition; Worth Publishers, Inc. NY, N.Y. (1975), pp. 71 77] as set out below.

**Table II - Conservative Substitutions**

| SIDE CHAIN CHARACTERISTIC | | AMINO ACID | | | | |
|---|---|---|---|---|---|---|
| Non-polar (hydrophobic) | | | | | | |
| A. | Aliphatic: | A | L | I | V | P |
| B. | Aromatic: | F | W | | | |
| C. | Sulfur-containing: | M | | | | |
| D. | Borderline: | G | | | | |

| Uncharged-polar | | | | | | |
|---|---|---|---|---|---|---|
| A. | Hydroxyl: | S | T | Y | | |
| B. | Amides: | N | Q | | | |
| C. | Sylfhydryl: | C | | | | |
| D. | Borderline: | G | | | | |
| Positively Charged (Basic): | | K | R | H | | |
| Negatively Charged (Acidic) | | D | E | | | |

And still other alternative, exemplary conservative substitutions are set out below.

**Table III - Conservative Substitutions**

| Original Residue | Exemplary Substitution |
|---|---|
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

It should be understood that the definition of peptides or polypeptides of the description is intended to include polypeptides bearing modifications other than insertion, deletion, or substitution of amino acid residues. By way of example, the modifications may be covalent in nature, and include for example, chemical bonding with polymers, lipids, other organic and inorganic moieties. Such derivatives may be prepared to increase circulating half-life of a polypeptide, or may be designed to improve the targeting capacity of the polypeptide for desired cells, tissues or organs. Similarly, the description further embraces IL4i1 peptides that have been covalently modified to include one or more water-soluble polymer attachments such as polyethylene glycol, polyoxyethylene glycol or polypropylene glycol.

Chemically modified peptide compositions in which the IL4i1 protein is linked to a polymer are included within the scope of the present invention. The polymer may be water soluble to prevent precipitation of the protein in an aqueous environment, such as a physiological environment. Suitable water-soluble polymers may be selected from the group consisting of, for example, polyethylene glycol (PEG), monomethoxypolyethylene glycol, dextran, cellulose, or other carbohydrate based polymers, poly-(N-vinyl pyrrolidone) polyethylene glycol, polypropylene glycol homopolymers, a polypropylene oxide/ethylene oxide copolymer polyoxyethylated polyols (e.g., glycerol) and polyvinyl alcohol. The selected polymer is usually modified to have a single reactive group, such as an active ester for acylation or an aldehyde for alkylation, so that the degree of polymerization may be controlled. Polymers may be of any molecular weight, and may be branched or unbranched, and mixtures of such polymers may also be used. When the chemically modified peptides are destined for therapeutic use, pharmaceutically acceptable polymers may be selected for use.

When the polymer is to be modified by an acylation reaction, the polymer should have a single reactive ester group. Alternatively, if the polymer is to be modified by reductive alkylation, the polymer should have a single reactive aldehyde group. A preferred reactive aldehyde is polyethylene glycol propionaldehyde, which is water stable, or mono CI-CIO alkoxy or aryloxy derivatives thereof (see U.S. Pat. No. 5,252,714).

Pegylation of Il4i1 peptides or variants thereof may be carried out by any of the pegylation reactions known in the art, as described, for example, in the following references: Focus on Growth Factors 3, 4 10 (1992); EP 0 154 316; and EP 0 401 384. Preferably, the pegylation is carried out via an acylation reaction or an alkylation reaction with a reactive polyethylene glycol molecule (or an analogous reactive water-soluble polymer). A preferred water-soluble polymer for pegylation of polypeptides is polyethylene glycol (PEG), including, but not limited to bi-functional PEGs. As used herein, "polyethylene glycol" is meant to encompass any of the forms of PEG that have been used to derivatize other proteins, such as mono (CI-CIO) alkoxy- or aryloxy-polyethylene glycol.

Chemical derivatization of the IL4i1 peptides may be performed under any suitable conditions used to react a biologically active substance with an activated polymer molecule. Methods for preparing pegylated peptide will generally comprise the steps of (a) reacting the polypeptide with polyethylene glycol, such as a reactive ester or aldehyde derivative of PEG, under conditions whereby the IL4i1 polypeptide becomes attached to one or more PEG groups, and (b) obtaining the reaction products. It will be apparent to one of ordinary skill in the art to select the optimal reaction conditions or the acylation reactions based on known parameters and the desired result.

Pegylated and other polymer modified polypeptides may generally be used to treat conditions that may be alleviated or modulated by administration of the IL4i1 polypeptides described herein. However, the chemically-derivatized polymer: IL4i1 polypeptide molecules disclosed herein may have additional activities, enhanced or reduced biological activity, or other characteristics, such as increased or decreased half-life, as compared to the nonderivatized molecules. The IL4i1 polypeptides, fragments thereof, variants and derivatives, may be employed alone, together, or in combination with other pharmaceutical compositions. For example, cytokines, growth factors, antibiotics, anti-inflammatories and/or chemotherapeutic agents may be coadministered as is appropriate for the indication being treated.

The present description provides compositions comprising purified polypeptides of the description. Preferred compositions comprise, in addition to the polypeptide of the description, a pharmaceutically acceptable (i.e., sterile and non-toxic) liquid, semisolid, or solid diluent that serves as a pharmaceutical vehicle, excipient or medium. Any diluent known in the art may be used. Exemplary diluents include, but are not limited to, water, saline solutions, polyoxyethylene sorbitan monolaurate, magnesium stearate, methyl- and propylhydroxybenzoate, talc, alginates, starches, lactose, sucrose, dextrose, sorbitol, mannitol, glycerol, calcium phosphate, mineral oil and cocoa butter.

In one embodiment, the description provides fusion proteins comprising at least a first and a second fusion peptide. The fusion partners are, generally speaking, covalently bonded to one another *via* a typical amine bond between the fusion peptides, thus creating one contiguous amino acid chain. In one specific embodiment, the first peptide of the fusion protein comprises a peptide with an amino acid sequence at least 75%, 80%, 85%, 86%, 87%, 88%, 89%, 90%, 91%, 92%, 93%, 94%, 95%, 96%, 97%, 98%, 99% or 100% identical to the amino acid sequence of SEQ ID NO:1 or 2. In one specific embodiment, the first fusion domain of the fusion peptide comprises an amino acid sequence at least 95% identical to the amino acid sequence of SEQ ID NO:1 or 2. In further embodiments, the first fusion domain comprises an amino acid sequence 100% identical to the amino acid sequence of SEQ ID NO:1 or 2.

Other types of fusion proteins provided by the present description include but are not limited to, fusions with secretion signals and other heterologous functional regions. Thus, for instance, a region of additional amino acids, particularly charged amino acids, may be added to the N-terminus of the protein to improve stability and persistence in the host cell, during purification or during subsequent handling and storage.

Additional fusion proteins include fusions for enhancing translocation of the protein across cell membranes. For example, Tat is an 86-amino acid protein involved in the replication of human immunodeficiency virus type 1 (HIV-1). The HIV-1 Tat transactivation protein is efficiently taken up by cells, and it has been demonstrated that low concentrations (nM) are sufficient to transactivate a reporter gene expressed from the HIV-1 promoter. Exogenous Tat protein is able to translocate through the plasma membrane and reach the nucleus to transactivate the viral genome. Tat peptide-mediated cellular uptake and nuclear translocation have been demonstrated in several systems. Chemically coupling a Tat-derived peptide (residues 37-72 of Tat) to several proteins results in their internalization in several cell lines or tissues (Fawell, et al., Proc Natl Acad Sci USA 91:664-668, 1994; Anderson, et al., Biochem Biophys Res Commun 194:876-8884, 1993; Fahraeus, et al., Curr Biol 6:84-91, 1996; Nagahara, et al., Nat Med 4:1449-1452, 1998).

It is well-known that a region of the Tat protein centered on a cluster of basic amino acids is responsible for this translocation activity. A synthetic peptide consisting of the Tat basic amino acids 48-60 with a cysteine residue at the C-terminus coupled to fluorescein maleimide translocates to the cell nucleus as determined by fluorescence microscopy. In addition, a fusion protein (Tat-NLS-β-Gal) consisting of Tat amino acids 48-59 fused by their amino-terminus to β-gatactosidase amino acids 9-1023 translocates to the cell nucleus in an ATP-dependent, cytosolic factor-independent manner. Accordingly, the fusion proteins of the present description may comprise all or a portion of HIV-Tat, such as any sequential residues of the Tat protein basic peptide motif 37-72 (37-CFITKALGISYGRKKRRQRRRPPQGSQTHQVSLSKQ-72 (SEQ ID NO:3). The minimum number of amino acid residues can be in the range of from three to six. In one embodiment, the Tat portion of the fusion protein is from three to five contiguous amino acids in length. In another embodiment, the Tat portion of the fusion protein is four amino acids in length, i.e., the minimal requirement for one alpha helical turn. In another embodiment, the Tat portion of the fusion protein comprises Tat protein residues 48-57 (GRKKRRQRRR) (SEQ ID NO:4).

In additional embodiments of fusion proteins, a region may be added to facilitate purification. For example, "histidine tags" ("his tags") or "lysine tags" (the second fusion peptide) may be appended to the first fusion peptide. Examples of histidine tags include, but are not limited to hexaH, heptaH and hexaHN. Additional examples of purification tags are disclosed in Waugh, D.S., Trends in Biotechnology, 23(6):316-320 (June 2005), and Gaberc-Porekar V. and Menart, V., J. Biochem. Biophys. Methods. 49:335-360 (2001). Examples of lysine tags include, but are not limited to pentaL, heptaL and FLAG. Additional examples of solubility tags are also disclosed in Waugh, D.S., Trends in Biotechnology, 23(6) 316-320 (June 2005). Such regions may be removed prior to final preparation of the protein. Other examples of a second fusion peptide include, but are not limited to, glutathione S-transferase (GST) and alkaline phosphatase (AP).

The addition of peptide moieties to proteins, whether to engender secretion or excretion, to improve stability and to facilitate purification or translocation, among others, is a familiar and routine technique in the art and may include modifying amino acids at the terminus to accommodate the tags. For example in SEQ ID NO: 2, the N-terminus amino acid may be modified to, for example, arginine and/or serine to accommodate a tag. Of course, the amino acid residues of the C-terminus may also be modified to accommodate tags. One particularly useful fusion protein comprises a heterologous region from immunoglobulin that can be used solubilize proteins. For example, EP A0464 533 discloses fusion proteins comprising various portions of constant region of immunoglobin molecules together with another human protein or part thereof. In many cases, the Fc part in a fusion protein is thoroughly advantageous for use in therapy and diagnosis and thereby results, for example, in improved pharmacokinetic properties (EP A0232 262). On the other hand, for some uses, it would be desirable to be able to delete the Fc part after the fusion protein has been expressed, detected and purified in the advantageous manner described.

The fusion proteins of the current description can be recovered and purified from recombinant cell cultures by well-known methods including, but not limited to, ammonium sulfate or ethanol precipitation, acid extraction, anion or cation exchange chromatography, phosphocellulose chromatography, hydrophobic interaction chromatography, affinity chromatography, e.g., immobilized metal affinity chromatography (IMAC), hydroxylapatite chromatography and lectin chromatography. High performance liquid chromatography ("HPLC") may also be employed for purification. Well-known techniques for refolding protein may be employed to regenerate active conformation when the fusion protein is denatured during isolation and/or purification.

Fusion proteins of the present description include, but are not limited to, products of chemical synthetic procedures and products produced by recombinant techniques from a prokaryotic or eukaryotic host, including, for example, bacterial, yeast, higher plant, insect and mammalian cells. Depending upon the host employed in a recombinant production procedure, the fusion proteins of the present description may be glycosylated or may be non-glycosylated. In addition, fusion proteins of the description may also include an initial modified methionine residue, in some cases as a result of host-mediated processes.

The description also relates to isolated nucleic acids and to constructs comprising these nucleic acids. The nucleic acids of the description can be DNA or RNA, for example, mRNA. The nucleic acid molecules can be double-stranded or single-stranded; single stranded RNA or DNA can be the coding, or sense, strand or the non-coding, or antisense, strand. In particular, the nucleic acids may encode any polypeptide of the description, including, but not limited to, the fusion proteins of the present description. For example, the nucleic acids of the description include polynucleotide sequences that encode glutathione-S-transferase (GST) fusion protein, poly-histidine (e.g., His6), poly-HN, poly-lysine, hemagglutinin, HSV-Tag and at least a portion of HIV-Tat. If desired, the nucleotide sequence of the isolated nucleic acid can include additional non-coding sequences such as non-coding 3' and 5' sequences (including regulatory sequences, for example).

The nucleic acid molecules of the description can be "isolated." As used herein, an "isolated" nucleic acid molecule or nucleotide sequence is intended to mean a nucleic acid molecule or nucleotide sequence that is not flanked by nucleotide sequences normally flanking the gene or nucleotide sequence (as in genomic sequences) and/or has been completely or partially removed from its native environment (e.g., a cell, tissue). For example, nucleic acid molecules that have been removed or purified from cells are considered isolated. In some instances, the isolated material will form part of a composition (for example, a crude extract containing other substances), buffer system or reagent mix. In other circumstances, the material may be purified to near homogeneity, for example as determined by PAGE or column chromatography such as HPLC. Thus, an isolated nucleic acid molecule or nucleotide sequence can includes a nucleic acid molecule or nucleotide sequence which is synthesized chemically, using recombinant DNA technology or using any other suitable method. To be clear, a nucleic acid contained in a vector would be included in the definition of "isolated" as used herein. Also, isolated nucleotide sequences include recombinant nucleic acid molecules (e.g., DNA, RNA) in heterologous organisms, as well as partially or substantially purified nucleic acids in solution. "Purified," on the other hand is well understood in the art and generally means that the nucleic acid molecules are substantially free of cellular material, cellular components, chemical precursors or other chemicals beyond, perhaps, buffer or solvent. "Substantially free" is not intended to mean that other components beyond the novel nucleic acid molecules are undetectable. The nucleic acid molecules of the present description may be isolated or purified. Both in vivo and in vitro RNA transcripts of a DNA molecule of the present description are also encompassed by "isolated" nucleotide sequences.

The description also encompasses variations of the nucleotide sequences of the description, such as those encoding functional fragments or variants of the polypeptides as described above. Such variants can be naturally-occurring, or non-naturally-occurring, such as those induced by various mutagens and mutagenic processes. Intended variations include, but are not limited to, addition, deletion and substitution of one or more nucleotides which can result in conservative or non-conservative amino acid changes, including additions and deletions.

The description described herein also relates to fragments of the isolated nucleic acid molecules described herein. The term "fragment" is intended to encompass a portion of a nucleotide sequence described herein which is from at least 20 contiguous nucleotides to at least 50 contiguous nucleotides or longer in length. Such fragments may be useful as probes and primers. In particular, primers and probes may selectively hybridize to the nucleic acid molecule encoding the polypeptides described herein. For example, fragments which encode polypeptides that retain activity, as described below, are particularly useful.

The description also provides nucleic acid molecules that hybridize under high stringency hybridization conditions, such as for selective hybridization, to the nucleotide sequences described herein (e.g., nucleic acid molecules which specifically hybridize to a nucleotide sequence encoding polypeptides described herein and encode an IL4i1 or variant described herein). Hybridization probes include synthetic oligonucleotides which bind in a base-specific manner to a complementary strand of nucleic acid. Suitable probes include polypeptide nucleic acids, as described in Nielsen et al., Science, 254:1497-1500 (1991).

Such nucleic acid molecules can be detected and/or isolated by specific hybridization e.g., under high stringency conditions. "Stringency conditions" for hybridization is a term of art that refers to the incubation and wash conditions, e.g., conditions of temperature and buffer concentration, which permit hybridization of a particular nucleic acid to a second nucleic acid; the first nucleic acid may be perfectly complementary, i.e., 100%, to the second, or the first and second may share some degree of complementarity, which is less than perfect, e.g., 60%, 75%, 85%, 95% or more. For example, certain high stringency conditions can be used which distinguish perfectly complementary nucleic acids from those of less complementarity.

"High stringency conditions", "moderate stringency conditions" and "low stringency conditions" for nucleic acid hybridizations are explained in Current Protocols in Molecular Biology, John Wiley & Sons, (1998)). The exact conditions which determine the stringency of hybridization depend not only on ionic strength, e.g., 0.2 X SSC, 0.1 X SSC of the wash buffers, temperature, e.g., room temperature, 42°C., 68°C., etc., and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, high, moderate or low stringency conditions may be determined empirically.

By varying hybridization conditions from a level of stringency at which no hybridization occurs to a level at which hybridization is first observed, conditions which will allow a given sequence to hybridize with the most similar sequences in the sample can be determined.

The nucleic acids described herein can be amplified by methods known in the art. For example, amplification can be accomplished by the polymerase chain reaction (PCR). See PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Eckert et al., PCR Methods and Applications 1:17 (1991); PCR (eds. McPherson et al., IRL Press, Oxford); and U.S. Pat. No. 4,683,202. Other suitable amplification methods include the ligase chain reaction (LCR) (see Wu and Wallace, Genomics, 4:560 (1989), Landegren et al., Science, 241:1077 (1988)), transcription amplification (Kwoh et al., Proc. Natl. Acad. Sci. USA, 86:1173 (1989)), and self-sustained sequence replication (Guatelli et al., Proc. Nat. Acad. Sci. USA, 87:1874 (1990)) and nucleic acid based sequence amplification (NASBA).

The present description also relates to vectors that include nucleic acid molecules of the present description, host cells that are genetically engineered with vectors of the description and the production of proteins of the description by recombinant techniques.

In accordance with this aspect of the description, the vector may be, for example, a plasmid vector, a single-or double-stranded phage vector, or a single-or double-stranded RNA or DNA viral vector. Such vectors may be introduced into cells as polynucleotides, for example DNA, by well-known techniques for introducing DNA and RNA into cells. Viral vectors may be replication competent or replication defective. In the latter, case viral propagation generally will occur only in complementing host cells.

In certain respects, the vectors to be used are those for expression of polynucleotides and proteins of the present invention. Generally, such vectors comprise cis-acting control regions effective for expression in a host operatively linked to the polynucleotide to be expressed. Appropriate trans-acting factors are supplied by the host, supplied by a complementing vector or supplied by the vector itself upon introduction into the host.

A great variety of expression vectors can be used to express the proteins of the invention. Such vectors include chromosomal, episomal and virus-derived vectors, e.g., vectors derived from bacterial plasmids, from bacteriophage, from yeast episomes, from yeast chromosomal elements, from viruses such as adeno-associated virus, lentivirus, baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. All may be used for expression in accordance with this aspect of the present invention. Generally, any vector suitable to maintain, propagate or express polynucleotides or proteins in a host may be used for expression in this regard.

The DNA sequence in the expression vector is operatively linked to appropriate expression control sequence(s) including, for instance, a promoter to direct mRNA transcription. Representatives of such promoters include, but are not limited to, the phage lambda PL promoter, the E. coli lac, trp and tac promoters, HIV promoters, the SV40 early and late promoters and promoters of retroviral LTRs, to name just a few of the well-known promoters. In general, expression constructs will contain sites for transcription, initiation and termination and, in the transcribed region, a ribosome binding site for translation. The coding portion of the mature transcripts expressed by the constructs will include a translation initiating AUG at the beginning and a termination codon (UAA, UGA or UAG) appropriately positioned at the end of the polypeptide to be translated.

In addition, the constructs may contain control regions that regulate, as well as engender expression. Generally, such regions will operate by controlling transcription, such as repressor binding sites and enhancers, among others.

Vectors for propagation and expression generally will include selectable markers. Such markers also may be suitable for amplification or the vectors may contain additional markers for this purpose. In this regard, the expression vectors may contain one or more selectable marker genes to provide a phenotypic trait for selection of transformed host cells. Preferred markers include dihydrofolate reductase or neomycin resistance for eukaryotic cell culture, and tetracycline, kanamycin or ampicillin resistance genes for culturing E. coli and other bacteria.

The vector containing the appropriate DNA sequence, as well as an appropriate promoter, and other appropriate control sequences, may be introduced into an appropriate host using a variety of well-known techniques suitable to expression therein of a desired polypeptide. Representative examples of appropriate hosts include bacterial cells, such as, but not limited to, E. coli, Streptomyces, Bacillus, and Salmonella cells; fungal cells, such as yeast cells; insect cells such as Drosophila S2 and Spodoptera Sf9 cells; animal cells such as CHO, COS and Bowes melanoma cells; and plant cells. Hosts for of a great variety of expression constructs are well known, and those of skill in the art will be enabled by the present disclosure to select an appropriate host for expressing one of the proteins of the present description.

Examples of vectors that may be useful for fusion proteins include, but are not limited to, pGEX (Pharmacia Biotech Inc; Smith and Johnson (1988) Gene 67, 31 40), pMAL (New England Biolabs, Beverly, Mass.) and pRIT5 (Pharmacia, Piscataway, N.J.) that fuse glutathione-S-transferase (GST), maltose E binding protein, or protein A, respectively, to the target recombinant protein. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Examples of vectors for expression in yeast S. cerevisiae include pYepSecl (Baldari, et al, (1987) EMBO J. 6, 229 234), pMFa (Kurjan and Herskowitz (1982) Cell 30, 933 943), pJRY88 (Schultz et al., (1987) Gene 54, 113 123), pYES2 (Invitrogen Corporation, San Diego, Calif., and picZ (InVitrogen Corp, San Diego, Calif.).

Alternatively, the IL4i1 proteins and/or variants thereof can be expressed in insect cells using baculovirus expression vectors. Baculovirus vectors available for expression of proteins in cultured insect cells (e.g., SF9 cells) include the pAc series (Smith et al, (1983) Mol. Cell. Biol. 3, 2156 2165) and the pVL series (Lucklow and Summers (1989) Virology 170, 31 39).

In yet another embodiment, a nucleic acid of the description is expressed in mammalian cells using a mammalian expression vector. Examples of mammalian expression vectors include pCDM8 (Seed (1987) Nature 329, 840) and pMT2PC (Kaufman et al. (1987) EMBO J. 6, 187 195). When used in mammalian cells, the expression vector's control functions are often provided by viral regulatory elements. For example, commonly used promoters are derived from polyoma, adenovirus 2, cytomegalovirus and Simian Virus 40. For other suitable expression systems for both prokaryotic and eukaryotic cells, see, e.g., Chapters 16 and 17 of Sambrook et al., (Eds.) Molecular Cloning: A Laboratory Manual. 2nd Ed., Cold Spring Harbor Laboratory, Cold Spring Harbor Laboratory Press, Cold Spring Harbor, N.Y., 1989 and later editions.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include liver-specific promoters (e.g., albumin promoter), lymphoid-specific promoters such as, but not limited to, T cell receptors and immunoglobulins, and neuron-specific promoters (e.g., neurofilament promoter) to name a few. Developmentally-regulated promoters are also encompassed, e.g., the murine hox promoters (Kessel and Gruss (1990) Science 249, 374 379) and the α-fetoprotein promoter (Campes and Tilghman (1989) Genes Dev. 3, 537 546).

The present description also relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. The host cell can be stably or transiently transfected with the construct. The polynucleotides may be introduced alone or with other polynucleotides. Such other polynucleotides may be introduced independently, co-introduced or introduced joined to the polynucleotides of the description. As used herein, a "host cell" is a cell that normally does not contain any of the nucleotides of the present description and contains at least one copy of the nucleotides of the present description. Thus, a host cell as used herein can be a cell in a culture setting or the host cell can be in an organism setting where the host cell is part of an organism, organ or tissue.

Suitable host cells for expression of the polypeptides of the description include, but are not limited to, prokaryotes, yeast, and eukaryotes. If a prokaryotic expression vector is employed, then the appropriate host cell would be any prokaryotic cell capable of expressing the cloned sequences. Suitable prokaryotic cells include, but are not limited to, bacteria of the genera Escherichia, Bacillus, Salmonella, Pseudomonas, Streptomyces and Staphylococcus.

If a eukaryotic expression vector is employed, then the appropriate host cell would be any eukaryotic cell capable of expressing the cloned sequence. In one embodiment, eukaryotic cells are cells of higher eukaryotes. Suitable eukaryotic cells include, but are not limited to, non-human mammalian tissue culture cells and human tissue culture cells. Other host cells include, but are not limited to, insect cells, HeLa cells, Chinese hamster ovary cells (CHO cells), African green monkey kidney cells (COS cells), human 293 cells, and murine 3T3 fibroblasts. Propagation of such cells in cell culture has become a routine procedure (see, Tissue Culture, Academic Press, Kruse and Patterson, Eds. (1973)).

In addition, a yeast cell may be employed as a host cell. Yeast cells include, but are not limited to, the genera Saccharomyces, Pichia and Kluveromyces. In one embodiment, the yeast hosts are S. cerevisiae or P. pastoris. Yeast vectors may contain an origin of replication sequence from a 2T yeast plasmid, an autonomously replication sequence (ARS), a promoter region, sequences for polyadenylation, sequences for transcription termination and a selectable marker gene. Shuttle vectors for replication in both yeast and E. coli are also included herein.

Alternatively, insect cells may be used as host cells. In one embodiment, the polypeptides of the description are expressed using a baculovirus expression system (see, Luckow et al., Bio/Technology, 1988, 6, 47; BACULOVIRUS EXPRESSION VECTORS: A LABORATORY MANUAL, O'Rielly et al. (Eds.), W.H. Freeman and Company, New York, 1992; and U.S. Pat. No. 4,879,236). In addition, commercially available complete baculovirus expression systems can, for example, be used for production in insect cells.

Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Introduction of a construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods in Molecular Biology (1986).

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook, et al (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. To identify and select these integrants, a gene that encodes a selectable marker (e.g., resistance to antibiotics) is generally introduced into the host cells along with the gene of interest. Various selectable markers include those that confer resistance to drugs, such as G418, hygromycin, dihydrofolate reductase (DHFR) and methotrexate. Nucleic acid encoding a selectable marker can be introduced into a host cell on the same vector as that encoding peptides of the present description or can be introduced on a separate vector. Cells stably transfected with the introduced nucleic acid can be identified by drug selection (e.g., cells that have incorporated the selectable marker gene will survive, while the other cells die).

In addition to the above-mentioned promoters, the expression vectors may contain enhancers, splicing signals, polyadenylation signals, selectable markers and an SV40 replication origin. Suitable selectable markers include, but are not limited to the dihydrofolate reductase (DHFR) gene which provides resistance to methotrexate (MTX), the ampicillin resistance gene, and the neomycin resistance gene.

Examples of the expression vectors each containing the DNA coding for the IL4i1 protein, or portions, fragments and soluble constructs thereof, include the vector (such as one described above), into which a promoter is operably linked to the nucleotide sequence encoding the desired IL4i1 construct, a polyadenylation signal downstream from the nucleotide sequence encoding the IL4i1 construct, and, optionally, an operable DHFR gene.

The IL4i1 expression vectors prepared as above are introduced into host cells by any known method, including, but not limited to the calcium phosphate method and electroporation.

Transformants carrying the expression vectors are selected based on the above-mentioned selectable markers. Repeated clonal selection of the transformants using the selectable markers allows selection of stable cell lines expressing the IL4i1 constructs. Increased MTX concentrations in the selection medium allows gene amplification and greater expression of the desired IL4i1. The host cells, for example CHO cells, containing the recombinant IL4i1 or variant thereof can be produced by cultivating the CHO cells containing the IL4i1 (or variant thereof) expression vectors constitutively expressing the IL4i1 or variant thereof.

Accordingly, the current description also relates to methods of producing an IL4i1 or variant thereof comprising culturing the host cells of the description under conditions such that the Il4i1 or variant thereof is expressed, and recovering said protein. The culture conditions required to express the proteins of the current description are dependent upon the host cells that are harboring the polynucleotides of the current invention. The culture conditions for each cell type are well-known in the art and can be easily optimized, if necessary. For example, a nucleic acid encoding a polypeptide of the description, or a construct comprising such nucleic acid, can be introduced into a suitable host cell by a method appropriate to the host cell selected, e.g., transformation, transfection, electroporation, infection, such that the nucleic acid is operably linked to one or more expression control elements as described herein. Host cells can be maintained under conditions suitable for expression in vitro or in vivo, whereby the encoded polypeptide is produced. For example host cells may be maintained in the presence of an inducer, suitable media supplemented with appropriate salts, growth factors, antibiotic, nutritional supplements, etc., which may facilitate protein expression. In additional embodiments, the IL4i1 or variant thereof of the invention can be produced by in vitro translation of a nucleic acid that encodes the IL4i1 or variant thereof, by chemical synthesis or by any other suitable method. If desired, the IL4i1 or variant thereof can be isolated from the host cell or other environment in which the protein is produced or secreted. It should therefore be appreciated that the methods of producing the Il4i1 or variants thereof encompass expression of the polypeptides in a host cell of a transgenic animal or plant. See U.S. Pat. Nos. 6,013,857, 5,990385, and 5,994,616.

In situations where the Il4i1 or variants thereof will be found primarily intracellularly, intracellular material (including inclusion bodies for Gram-negative bacteria) can be extracted from the host cell using any standard technique known to one of ordinary skill in the art. Such methods would encompass, by way of example and not by way of limitation, lysing the host cells to release the contents of the periplasm/cytoplasm by French press, homogenization and/or sonication followed by centrifugation.

If the Il4i1 or variant thereof has formed inclusion bodies in the cytosol, such inclusion bodies may frequently bind to the inner and/or outer cellular membranes. Upon centrifugation, the inclusion bodies will be found primarily in the pellet material. The pellet material can then be treated at pH extremes or with one or more chaotropic agents such as a detergent, guanidine, guanidine derivatives, urea, or urea derivatives in the presence of a reducing agent such as dithiothreitol at alkaline pH or tris-carboxyethyl phosphine at acid pH to release, break apart and solubilize the inclusion bodies. Once solubilized, the IL4i1 or variant thereof can be analyzed using gel electrophoresis, immunoprecipitation or the like. Various methods of isolating the IL4i1 or variants thereof would be apparent to one of ordinary skill in the art, for example, isolation may be accomplished using standard methods such as those set forth below and in Marston et al (1990) Meth. Enzymol. 182, 264 275.

If the IL4i1 or variant thereof is not biologically active following the isolation procedure employed, various methods for "refolding" or converting the polypeptide to its tertiary structure and generating disulfide linkages, can be used to restore biological activity. Methods known to one of ordinary skill in the art include adjusting the pH of the solubilized polypeptide to a certain pH, usually above 7, and in the presence of a particular concentration of a chaotrope. The selection of chaotrope is very similar to the choices used for inclusion body solubilization, but usually at a lower concentration, and is not necessarily the same chaotrope as used for the solubilization. It may be required to employ a reducing agent or the reducing agent plus its oxidized form in a specific ratio, to generate a particular redox potential allowing for disulfide shuffling during the formation of the protein's cysteine bridge(s). Some of the commonly used redox couples include cysteine/cysteine, glutathione (GSH)/dithiobis GSH, cupric chloride, dithiothreitol (DTT)/dithiane DTT, 2-mercaptoethanol (bME)/dithio-b(ME). To increase the efficiency of the refolding, it may be necessary to employ a cosolvent, such as glycerol, polyethylene glycol of various molecular weights and arginine.

Regardless of method of preparation, be it recombinantly or synthetically, the IL4i1 peptides and variants thereof can be prepared as a composition. In one embodiment, the composition is a pharmaceutical composition. For example, one or more cofactors may be added to the IL4i1 peptides and variants thereof to form a composition. Cofactors that may be added include, but are not limited to, heparin, hyaluronic acid, a fibronectin, an elastin, a laminin, albumin, a proteoglycan, collagen, gelatin, a divalent cation, calcium chloride, zinc sulfate, magnesium chloride, sodium bicarbonate, sodium chloride, sodium acetate, or sodium phosphate. In some embodiments, a protein or a protein fragment may be added as a cofactor to the IL4i1 peptides and variants thereof.

The compositions, or pharmaceutical compositions, comprising the nucleic acid molecules or polypeptides typically comprise the nucleic acid molecule or protein and a pharmaceutically acceptable carrier. As used herein, "pharmaceutically acceptable carrier" or "pharmaceutical carrier" is intended to include any and all solvents, dispersion media, coatings, antibacterial and antifungal agents, isotonic and absorption delaying agents, and the like, compatible with pharmaceutical administration. The nature of the pharmaceutical carrier or other ingredients will depend on the specific route of administration and particular embodiment of the invention to be administered. Examples of techniques and protocols that are useful in this context are, inter alia, found in Remington: The Science and Practice of Pharmacy, Beringer et al. (Eds), 21st Ed., Lippincott Williams & Wilkins (2005). Examples of such pharmaceutical carriers or diluents include, but are not limited to, water, saline, Ringer's solution, dextrose solution and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include oral and parenteral (e.g., intravenous, intradermal, subcutaneous, inhalation, transdermal (topical), transmucosal and rectal administration). Solutions or suspensions used for parenteral, intradermal or subcutaneous application can include, but are not limited to, a sterile diluent such as water for injection, saline solution, fixed oils, polyethylene glycols, glycerine, propylene glycol or other synthetic solvents, antibacterial agents such as benzyl alcohol or methyl parabens, antioxidants such as ascorbic acid or sodium bisulfite, chelating agents such as ethylenediaminetetraacetic acid, buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable pharmaceutical carriers include physiological saline, bacteriostatic water, Cremophor ELTM (BASF, Parsippany, N.J.) or phosphate buffered saline (PBS). In all cases, the compositions must be sterile and should be fluid to the extent that easy syringeability exists. It must be stable under the conditions of manufacture and storage and must be preserved against the contaminating action of microorganisms such as bacteria and fungi. The pharmaceutical carrier can be a solvent or dispersion medium containing, for example, water, ethanol, polyol (for example, glycerol, propylene glycol and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it may be desirable to include isotonic agents, for example, sugars, polyalcohols such as manitol, sorbitol, sodium chloride in the composition. Prolonged absorption of the injectable compositions can be brought by including in the composition an agent which delays absorption, for example, aluminum monostearate and gelatin.

Sterile injectable solutions can be prepared by incorporating the active compound (e.g., the IL4i1 peptides and variants thereof) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutical carrier. They can be enclosed in gelatin capsules or compressed into tablets. For the purpose of oral therapeutic administration, the active compound can be incorporated with excipients and used in the form of tablets, troches or capsules. Oral compositions can also be prepared using a fluid pharmaceutical carrier for use as a mouthwash, wherein the compound in the fluid pharmaceutical carrier is applied orally and swished and expectorated or swallowed. Pharmaceutically compatible binding agents, and/or adjuvant materials can be included as part of the composition. The tablets, pills, capsules, troches and the like may contain any of the following ingredients, or compounds of a similar nature, such as but not limited to a binder, such as microcrystalline cellulose, gum tragacanth or gelatin, an excipient such as starch or lactose, a disintegrating agent such as alginic acid, Primogel or corn starch, a lubricant such as magnesium stearate or Sterotes, a glidant such as colloidal silicon dioxide, a sweetening agent such as sucrose or saccharin, or a flavoring agent such as peppermint, methyl salicylate or orange flavoring.

For administration by inhalation, the compounds are delivered in the form of an aerosol spray from pressured container or dispenser which contains a suitable propellant, e.g., a gas such as carbon dioxide or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels or creams as generally known in the art.

In one embodiment, the active compounds are prepared with pharmaceutical carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These compositions can be prepared according to methods known to those skilled in the art, for example, as described in U.S. Pat. No. 4,522,811. It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved.

The nucleic acid molecules of the invention can be inserted into vectors and used as gene therapy vectors. Gene therapy vectors can be delivered to a subject by any of a number of routes, e.g., as described in U.S. Pat. No. 5,703,055. Delivery can thus also include, e.g., intravenous injection, local administration (see U.S. Pat. No. 5,328,470) or stereotactic injection, e.g., Chen et al. (1994) Proc. Natl. Acad. Sci. USA 91, 3054 3057. The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

The pharmaceutical compositions can be included in a container, pack or dispenser together with instructions for administration.

The dosage of the compounds will depend on the disease state or condition to be treated and other clinical factors such as weight and condition of the human or animal and the route of administration of the compound. For treating human or animals, between approximately 0.005 mg/kg of body weight to 500 mg/kg of body weight of the compound can be administered. Therapy is typically administered at lower dosages and is continued until the desired therapeutic outcome is observed.

Methods of determining the dosages of compounds to be administered to a patient and modes of administering compounds to an organism are disclosed in U.S. application Ser. No. 08/702,282, filed Aug. 23, 1996, and International patent publication number WO 96/22976, published Aug. 1, 1996. Those skilled in the art will appreciate that such descriptions are applicable to the present invention and can be easily adapted to it.

The proper dosage depends on various factors such as the type of disease being treated, the particular composition being used and the size and physiological condition of the patient. Therapeutically effective doses for the compounds described herein can be estimated initially from cell culture and animal models. For example, a dose can be formulated in animal models to achieve a circulating concentration range that initially takes into account the IC50 as determined in cell culture assays. The animal model data can be used to more accurately determine useful doses in humans.

Plasma half-life and biodistribution of the active and metabolites in the plasma and major organs can also be determined to facilitate the selection of drugs most appropriate to inhibit a disorder. Such measurements can be carried out, for example, using BPLC analysis performed on the plasma of animals treated with the drug and the location of radiolabeled compounds can be determined using detection methods such as X-ray, CAT scan and MRI. Compositions that exhibit poor pharmacokinetic characteristics can be optimized by altering the chemical structure and retesting. In this regard, compounds displaying good pharmacokinetic characteristics can be used as a model.

Toxicity studies can also be carried out by measuring the blood cell composition. For example, toxicity studies can be carried out in a suitable animal model as follows: (1) the compound is administered to mice (an untreated control mouse should also be used); (2) blood samples are periodically obtained via the tail vein from one mouse in each treatment group; and (3) the samples are analyzed for red and white blood cell counts, blood cell composition and the percent of lymphocytes versus polymorphonuclear cells. A comparison of results for each dosing regime with the controls indicates if toxicity is present.

At the termination of each toxicity study, further studies can be carried out by sacrificing the animals. Representative animals from each treatment group can then be examined by gross necropsy for immediate evidence of metastasis, unusual illness or toxicity. Gross abnormalities in tissue are noted and tissues are examined histologically. Compounds causing a reduction in body weight or blood components are less preferred, as are compounds having an adverse effect on major organs. In general, the greater the adverse effect the less preferred the compound.

Agents, or modulators that have a stimulatory or inhibitory effect on activity of the IL4i1 peptides and variants thereof can be administered to individuals to treat (prophylactically or therapeutically) disorders or conditions. In conjunction with such treatment, the pharmacogenomics, i.e., the study of the relationship between an individual's genotype and that individual's response to a foreign compound or drug, of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents for prophylactic or therapeutic treatments based on a consideration of the individual's genotype. Such pharmacogenomics can further be used to determine appropriate dosages and therapeutic regimens. Accordingly, the activity of the IL4i1 peptides and variants thereof can be determined to direct selection of appropriate agent(s) for therapeutic or prophylactic treatment of the individual.

In one embodiment, the treatment methods of the present description comprise administering IL4i1 or a variant thereof to a subject that has been diagnosed with multiple sclerosis (MS) or Parkinson's Disease (PD) prior to the administration of the IL4i1 protein or variant thereof. In another embodiment, the treatment methods of the present description comprise administering IL4i1 or a variant thereof to a subject that has received a traumatic brain injury (TBI) or spinal cord injury (SCI) prior to the administration of the IL4i1 protein. In other embodiments, the treatment methods of the present description comprise administering IL4i1 or a variant thereof to a subject that has been diagnosed with an idiopathic inflammatory demyelinating disease.

The present description is also directed to methods of diagnosing a subject as having a predisposition to having a condition marked by an impairment of myelin regeneration in the central nervous system, with the method comprising determining activity or levels of IL4-i1 protein in the subject. A reduction in the activity or levels of IL4-i1 in the subject is indicative that the subject has a predisposition to having a condition marked by an impairment of myelin regeneration. In one specific embodiment, the activity levels of IL4-i1 are determined in activated macrophages obtained from the subject.

Method of assessing or determining activity or levels of IL4i1 in activated macrophages are well known in the art. For example, RT-PCR detects IL4i1 mRNA expression, western blots and ELISA detects IL4i1 protein expression, immunostaining using an antibody generated against IL4i1 detects IL4i1 protein expression in situ. Since IL4i1 is an L-amino acid oxidase, IL4i1 activity can be assessed by hydrogen peroxide formation when IL4i1 protein is mixed with L-phenylalanine.

The description is also directed to methods of monitoring the progression of conditions marked by an impairment of myelin formation in the CNS comprising assessing over time the levels or activity of IL4-i1 in the subject. When the levels or activities of IL4i1 fall or decrease over time, the subject can be diagnosed with or determined to have a condition marked by an impairment of myelin formation. In one specific embodiment, the activity levels of IL4-i1 are determined in activated macrophages obtained from the subject.

The examples herein are meant for illustrative purposes and are not intended to limit the scope of the invention.

### Examples

### Example 1

To identify potential genes involved in promoting remyelination, a microarray profile of CNS remyelination was analyzed. Bioinformatic analysis of differential gene expression over 3 post-lesion time points indicated that remyelination could be characterized by i) increased inflammation and oligodendrocyte precursor recruitment (5 days postlesion (dpl)), followed by ii) decreased inflammation and initiation of oligodendrocyte differentiation (14dpl), and iii) myelin maturation (28dpl).

It was discovered that IL4i1 was highly expressed at 14dpl, but barely detectable at 5dpl and 28dpl (p-value=0.0023) (Figure 1). Its distinctive expression profile during remyelination suggests that IL4i1 is transiently upregulated during remyelination, and may function to (1) suppress inflammation, (2) promote transition of macrophages from M1 to M2 phenotype, and (3) positively affect differentiation of oligodendrocyte progenitor cells.

To examine the expression pattern of IL4i1 during remyelination, focal demyelination was performed by injecting lysolecithin into the ventral spinal cord white matter of adult mice (3 months old), and collect spinal cord tissues during remyelination at 5, 15 and 30 days post lesion (dpl). A probe against the mouse IL4i1 transcript was generated and was used to perform *in situ* hybridization on focally demyelinated mouse spinal cord tissue sections. Result shows that IL4i1 is expressed around the lesioned white matter during CNS remyelination (Figure 2).

Expression of IL4i1 will also be assessed over at least 3 different timepoints by quantitative real time PCR (BioRad CFX96 Real-Time System), and western blot analysis using commercially available IL4i1 antibodies (Abcam) on dissected lesioned vs. unlesioned spinal cord tissues.

### Example 2

To determine if IL4i1 is expressed by M2 macrophages, PCR analysis was performed on cDNA samples from spleen tissue, untreated monocyte derived macrophage cell line (RAW247), and IL-13 stimulated RAW247 cells. IL-13 is known to induce the M2 macrophage phenotype. Result shows IL4i1 mRNA expression in IL-13 induced cells, suggesting that IL4i1 is associated with the M2 macrophage response (Figure 3).

To determine if M2 macrophages from microglia express IL4i1, IL4 or IL13 is added to primary microglia cultures to promote the M2 phenotype, and IL4i1 expression can be quantified by quantitative real time PCR. As controls, IL4i1 expression in untreated and LPS treated microglia are used. To determine if M2 macrophages secrete IL4i1, Western blot analysis is then performed on tissue culture media from IL4/IL13 induced M2 macrophages. To determine if M2 macrophages express Il4i1 in spinal cord tissues after lysolecithin-induced demyelination, commercially available antibodies is used to label M1 macrophages, *e.g.,* anti-iNOS Ab, and label M2 macrophages, *e.g.,* anti-Argl Ab, anti-YMl Ab in spinal cord tissues after *in situ* hybridization with the IL4i1 probe. To determine if IL4i1 expression in spinal cord lesions correlates with oligodendrocyte differentiation, lesioned tissues is co-labeled at least at 3 different timepoints for oligodendrocytes, e.g., anti-CCl Ab, OPCs, *e.g.,* anti-NKX2.2 Ab, and astrocytes, *e.g.,* anti-GFAP Ab.

### Example 3

To determine if IL4i1 directly promotes remyelination, a recombinant human IL4i1 (Origene) is added to culture media of *ex vivo* mouse cerebellar slice cultures following lysolecithin-induced demyelination]. Immunostaining I then performed to analyze IL4i1 treated vs. untreated cultures to quantify the extent of OPC proliferation (BrdU labeling and anti-NKX2.2), oligodendrocyte remyelination (anti-MBP), and macrophage polarization (anti-iNOS, anti-Argl, anti-YM1). To determine if IL4i1 regulates myelination either by directly (i) promoting oligodendrocyte differentiation/maturation, or (ii) attenuating inflammation, recombinant IL4i1 is added to primary OPC cultures vs. M1 macrophages. The extent of oligodendrocyte maturation is then assessed morphologically following MBP immunolabeling and quantitative real time PCR for possible increase of myelin associated transcripts, including but not limited to MBP, PLP and MOG. To determine if recombinant IL4i1 attenuates inflammation in M1 macrophages, real time PCR is performed to determine if genes associated with inflammation, including osteopontin, CXCL13, CD36, (all are highly expressed at 5dpl in the remyelination transcriptome) decrease with IL4i1 incubation.

### Example 4

Since IL4i1 is upregulated at the onset of OL differentiation during remyelination IL4i1 likely promotes remyelination. A recombinant mouse IL4i1 (rmIL4i1) is delivered by (1) direct injection into lesions, and by (2) systemic injection into demyelinated mice. It has previously been determined that OPCs migrate to lesions by 5dpl, that OL differentiation can be observed by 10dpl, and that many axons will be remyelinated by 20dpl. If IL4i1 positively regulates remyelination, then it would be expected that IL4i1 gain-of-function by rmIL4i1 delivery will accelerate OL differentiation and remyelination in lesions, *i.e.,* increased remyelination by 10dpl instead of 20dpl.

Co-injection of lysolecithin with rmIL4i1 or vehicle resulted in similar sized lesions at 5 and 10dpl (n=3 per timepoint) suggesting that rmIL4i1 co-injection does not interfere with lysolecithin-induced demyelination (Fig 4B). Importantly, a significant increase in the number of CC1+Olig2+ OL was found in the lesions of rmIL4i1 injected mice compared to controls at 5 and 10dpl. Moreover, the proportion of myelin basic protein (MBP) labeling corresponding to myelin membrane formation was significantly greater in lesions of rmIL4i1 injected mice compared to control. These results suggest that rmIL4i1 injection accelerated oligodendrocyte differentiation and maturation in lesions.

For direct rmIL4i1 delivery, rmIL4i1 or control 1XPBS is co-injected with the demyelinating agent lysolecithin into the mouse spinal cord white matter, and spinal cord lesions are analyzed at 5, 10 and 20dpl. The co-injection strategy allows the direct targeting of exogenous compounds precisely to the lysolecithin-affected spinal cord region, and has previously been shown to be effective in remyelination experiments. For systemic rmIL4i1 delivery, rmIL4i1 is injected intraperitoneally for 5 consecutive days after demyelination and the mice are sacrificed at 10 and 20dpl. To analyze OL lineage cells in lesions, co-immunostaining analysis of spinal cord sections is performed by using the following antibodies: anti-Olig2 (for OL lineage cells), anti-Nkx2.2 (for OPC), anti-CCl (for OL), and anti-MBP (for myelinating OL). For OPC/OL survival, the extent of apoptosis in OPC and OL is then analyzed in lesions by apoptosis (TUNEL) assay and immunostaining analysis of OL.

### Example 5

To determine if rmIL4i1 is able to stimulate H₂O₂ production, rmIL4i1 was added to RAW264.7 macrophage cells, and the conditioned media was analyzed for the generation of H₂O₂. Addition of rmIL4i1 induced significant H₂O₂ production in the culture media suggesting that rmIL4i1 is effective for the gain-of-function experiment.

## Claims

1. A composition for use in the treatment of multiple sclerosis or other condition marked by an impairment of myelin regeneration in the central nervous system (CNS) in a subject, said composition comprising interleukin-4 induced gene 1 (IL4-il) protein.

2. The composition for its use according to claim 1, wherein the subject has been diagnosed with multiple sclerosis (MS), Parkinson's Disease (PD), or has received a traumatic brain injury or a spinal cord injury prior to the administration of the IL4-i1 protein.

3. The composition for its use according to claim 1, wherein the subject has a neuro-inflammatory disorder causing impairment of myelin regeneration and/or axonal loss prior to the administration of the IL4-i1 protein.

4. The composition for its use according to claim 3, wherein the neuro-inflammatory disorder causes demyelination.

5. The composition for its use according to any of claims 1-4, wherein the IL4-i1 protein is administered directly to the site in the CNS that is in need of myelin formation.

6. The composition for its use according to any of claims 1-4, wherein the IL4-i1 protein is administered systemically and/or peripherally to the subject.

7. A method of diagnosing a subject as having a predisposition to having a condition marked by impairment of myelin regeneration in the central nervous system (CNS), the method comprising determining activity or levels of interleukin-4 induced gene 1 (IL4-il) protein in a sample from the subject, wherein a reduction in the activity or levels of IL4-i1 in a sample from the subject is indicative that the subject has a predisposition to having a condition marked by demyelination or an impairment of myelin regeneration.

8. The method of claim 7, wherein the activity or levels of IL4-i1 are determined in activated macrophages.

9. The method of claim 8, wherein the activated macrophages are M2 activated macrophages.

10. The method of any of claims 7-9, wherein the condition marked by an impairment of myelin regeneration in the CNS is multiple sclerosis.

11. The method of any of claims 7-9, wherein the condition marked by an impairment of myelin regeneration in the CNS is traumatic brain injury or spinal cord injury.

12. The method of any of claims 9-11, wherein the condition marked by impairment of myelin is demyelination.

13. A method of monitoring the progression in a subject of a condition marked by impairment of myelin regeneration in central nervous system (CNS) tissue and/or axonal loss, the method comprising determining for at least two time points activity levels of interleukin-4 induced gene 1 (IL4-il) protein in a sample from the subject, wherein a reduction in the activity or levels of IL4-i1 in a sample from the subject over time is indicative that the condition marked by an impairment of myelin regeneration is progressing in the subject and wherein an increase in the activity or levels of IL4-i1 in the subject over time is indicative that the condition marked by an impairment of myelin regeneration is regressing in the subject.

14. The method of claim 13, wherein the subject is receiving a treatment for the condition marked by an impairment of myelin regeneration and wherein a reduction in the activity or levels of IL4-i1 in a sample from the subject over time is indicative that the treatment is not effective in slowing the progression of the condition marked by an impairment of myelin regeneration in the subject.

15. The method of claim 13, wherein the subject is receiving a treatment for the condition marked by an impairment of myelin regeneration and wherein an increase in the activity or levels of IL4-i1 in a sample from the subject over time is indicative that the treatment is effective in slowing the progression of the condition marked by an impairment of myelin regeneration in the subject.

16. The method of any of claims 13-15, wherein the condition marked by an impairment of myelin is demyelination.

17. The method of claim 13, wherein the subject is not receiving treatment for the condition marked by an impairment of myelin regeneration prior to the determining the activity or levels of IL4-i1.

18. The method of any of claims 13-17, wherein the activity or levels of IL4-i1 are determined in activated macrophages.

## Patentansprüche

1. Zusammensetzung zur Verwendung bei der Behandlung von Multipler Sklerose oder eines anderen Zustands, der durch eine Beeinträchtigung der Myelinregeneration im Zentralnervensystem (ZNS) bei einem Subjekt gekennzeichnet sind, wobei die Zusammensetzung Interleukin-4-induziertes Gen 1 (IL4-i1) -Protein umfasst.

2. Zusammensetzung für ihre Verwendung nach Anspruch 1, wobei bei dem Subjekt vor der Verabreichung des IL4-i1 -Proteins Multiple Sklerose (MS), Parkinson-Krankheit (PD) diagnostiziert wurde oder es eine traumatische Hirnverletzung oder eine Rückenmarksverletzung erlitten hat.

3. Zusammensetzung für ihre Verwendung nach Anspruch 1, wobei das Subjekt vor der Verabreichung des IL4-i1-Proteins eine neuroentzündliche Erkrankung hat, die eine Beeinträchtigung der Myelinregeneration und/oder einen axonalen Verlust verursacht.

4. Zusammensetzung für ihre Verwendung nach Anspruch 3, wobei die neuroentzündliche Erkrankung eine Demyelinisierung verursacht.

5. Zusammensetzung für ihre Verwendung nach einem der Ansprüche 1-4, wobei das IL4-i1-Protein direkt an die Stelle im ZNS verabreicht wird, die eine Myelinbildung benötigt.

6. Zusammensetzung für ihre Verwendung nach einem der Ansprüche 1-4, wobei das IL4-i1-Protein dem Subjekt systemisch und/oder peripher verabreicht wird.

7. Verfahren zur Diagnose eines Subjekts mit einer Veranlagung zu einem Zustand, der durch eine Beeinträchtigung der Myelinregeneration im Zentralnervensystem (ZNS) gekennzeichnet ist, wobei das Verfahren das Bestimmen der Aktivität oder der Spiegel von Interleukin-4-induziertem Gen 1 (IL4-i1) -Protein in einer Probe von dem Subjekt umfasst, wobei eine Verringerung der Aktivität oder der Spiegel von IL4-i1 in einer Probe von dem Subjekt anzeigt, dass das Subjekt eine Veranlagung für einen Zustand hat, der durch Demyelinisierung oder eine Beeinträchtigung der Myelinregeneration gekennzeichnet ist.

8. Verfahren nach Anspruch 7, wobei die Aktivität oder die Spiegel von IL4-i1 in aktivierten Makrophagen bestimmt werden.

9. Verfahren nach Anspruch 8, wobei die aktivierten Makrophagen M2-aktivierte Makrophagen sind.

10. Verfahren nach einem der Ansprüche 7-9, wobei der Zustand, der durch eine Beeinträchtigung der Myelinregeneration im ZNS gekennzeichnet ist, Multiple Sklerose ist.

11. Verfahren nach einem der Ansprüche 7-9, wobei der Zustand, der durch eine Beeinträchtigung der Myelinregeneration im ZNS gekennzeichnet ist, eine traumatische Hirnverletzung oder eine Rückenmarksverletzung ist.

12. Verfahren nach einem der Ansprüche 9-11, wobei der Zustand, der durch eine Beeinträchtigung von Myelin gekennzeichnet ist, Demyelinisierung ist.

13. Verfahren zum Überwachen des Fortschreitens eines Zustands bei einem Subjekt, der durch eine Beeinträchtigung der Myelinregeneration im Gewebe des Zentralnervensystems (ZNS) und/oder einen axonalen Verlust gekennzeichnet ist,
wobei das Verfahren das Bestimmen von Aktivitätsspiegeln von Interleukin-4-induziertem Gen 1 (IL4-i1) -Protein in einer Probe des Subjekts für mindestens zwei Zeitpunkte umfasst, wobei eine Verringerung der Aktivität oder der Spiegel von IL4-i1 in einer Probe des Subjekts im Laufe der Zeit anzeigt, dass der Zustand, der durch eine Beeinträchtigung der Myelinregeneration gekennzeichnet ist, bei dem Subjekt fortschreitet, und wobei eine Zunahme der Aktivität oder der IL4-i1-Spiegel beim Subjekt im Laufe der Zeit anzeigt, dass der Zustand, der durch eine Beeinträchtigung der Myelinregeneration gekennzeichnet ist, beim Subjekt zurückgeht.

14. Verfahren nach Anspruch 13, wobei das Subjekt eine Behandlung für den Zustand erhält, der durch eine Beeinträchtigung der Myelinregeneration gekennzeichnet ist, und wobei eine Verringerung der Aktivität oder der Spiegel von IL4-i1 in einer Probe des Subjekts im Laufe der Zeit anzeigt, dass die Behandlung bei der Verlangsamung des Fortschreitens des Zustands, der durch eine Beeinträchtigung der Myelinregeneration bei dem Subjekt gekennzeichnet ist, nicht wirksam ist.

15. Verfahren nach Anspruch 13, wobei das Subjekt eine Behandlung für den Zustand erhält, der durch eine Beeinträchtigung der Myelinregeneration gekennzeichnet ist, und wobei eine Zunahme der Aktivität oder der Spiegel von IL4-i1 in einer Probe des Subjekts im Laufe der Zeit anzeigt, dass die Behandlung bei der Verlangsamung des Fortschreitens des Zustands, der durch eine Beeinträchtigung der Myelinregeneration bei dem Subjekt gekennzeichnet ist, wirksam ist.

16. Verfahren nach einem der Ansprüche 13-15, wobei der Zustand, der durch eine Beeinträchtigung von Myelin gekennzeichnet ist, Demyelinisierung ist.

17. Verfahren nach Anspruch 13, wobei das Subjekt keine Behandlung für den Zustand erhält, der durch eine Beeinträchtigung der Myelinregeneration gekennzeichnet ist, bevor die Aktivität oder die Spiegel von IL4-i1 bestimmt werden.

18. Verfahren nach einem der Ansprüche 13-17, wobei die Aktivität oder die Spiegel von IL4-i1 in aktivierten Makrophagen bestimmt werden.

## Revendications

1. Composition pour utilisation dans le traitement de la sclérose en plaques ou d'une autre maladie marquée par une altération de la régénération de la myéline dans le système nerveux central (SNC) chez un sujet, ladite composition comprenant une protéine IL4-i1 (interleukin-4 induced gene 1).

2. Composition pour son utilisation selon la revendication 1, dans laquelle le sujet a été diagnostiqué d'une sclérose en plaques (SP), de la maladie de Parkinson (MP), ou a reçu un traumatisme crânien ou une lésion médullaire avant l'administration de la protéine IL4-i1.

3. Composition pour son utilisation selon la revendication 1, dans laquelle le sujet présente un trouble neuro-inflammatoire entraînant une altération de la régénération de la myéline et/ou une perte axonale avant l'administration de la protéine IL4-i1.

4. Composition pour son utilisation selon la revendication 3, dans laquelle le trouble neuro-inflammatoire entraîne une démyélinisation.

5. Composition pour son utilisation selon l'une quelconque des revendications 1-4, dans laquelle la protéine IL4-i1 est administrée directement sur le site dans le SNC qui a besoin d'une formation de myéline.

6. Composition pour son utilisation selon l'une quelconque des revendications 1-4, dans laquelle la protéine IL4-i1 est administrée de manière systémique et/ou périphérique au sujet.

7. Procédé de diagnostic d'un sujet comme présentant une prédisposition à présenter un problème marqué par une altération de la régénération de la myéline dans le système nerveux central (SNC), le procédé comprenant la détermination de l'activité ou de niveaux de protéine IL4-i1 (interleukin-4 induced gene 1) dans un échantillon provenant du sujet, dans lequel une réduction de l'activité ou de niveaux d'IL4-i1 dans un échantillon provenant du sujet indique que le sujet présente une prédisposition à présenter un problème marqué par la démyélinisation ou une altération de la régénération de la myéline.

8. Procédé selon la revendication 7, dans lequel l'activité ou les niveaux d'IL4-i1 sont déterminés dans des macrophages activés.

9. Procédé selon la revendication 8, dans lequel les macrophages activés sont des macrophages activés M2.

10. Procédé selon l'une quelconque des revendications 7-9, dans lequel le problème marqué par une altération de la régénération de la myéline dans le SNC est une sclérose en plaques.

11. Procédé selon l'une quelconque des revendications 7-9, dans lequel le problème marqué par une altération de la régénération de la myéline dans le SNC est un traumatisme crânien ou une lésion médullaire.

12. Procédé selon l'une quelconque des revendications 9-11, dans lequel le problème marqué par une altération de la myéline est la démyélinisation.

13. Procédé de surveillance de la progression chez un sujet d'un problème marqué par une altération de la régénération de la myéline dans les tissus du système nerveux central (SNC) et/ou une perte axonale, le procédé comprenant la détermination pour au moins deux instants temporels de niveaux d'activité de la protéine IL4-i1 (interleukin-4 induced gene 1) dans un échantillon provenant du sujet, dans lequel une réduction de l'activité ou des niveaux d'IL4-i1 dans un échantillon provenant du sujet au fil du temps indique que le problème marqué par une altération de la régénération de la myéline progresse chez le sujet et dans lequel une augmentation de l'activité ou des niveaux d'IL4-i1 chez le sujet au fil du temps indique que le problème marqué par une altération de la régénération de la myéline régresse chez le sujet.

14. Procédé selon la revendication 13, dans lequel le sujet reçoit un traitement pour le problème marqué par une altération de la régénération de la myéline et dans lequel une réduction de l'activité ou des niveaux d'IL4-i1 dans un échantillon provenant du sujet au fil du temps indique que le traitement n'est pas efficace dans le ralentissement de la progression du problème marqué par une altération de la régénération de la myéline chez le sujet.

15. Procédé selon la revendication 13, dans lequel le sujet reçoit un traitement pour le problème marqué par une altération de la régénération de la myéline et dans lequel une augmentation de l'activité ou des niveaux d'IL4-i1 dans un échantillon provenant du sujet au fil du temps indique que le traitement est efficace dans le ralentissement de la progression du problème marqué par une altération de la régénération de la myéline chez le sujet.

16. Procédé selon l'une quelconque des revendications 13-15, dans lequel le problème marqué par une altération de la myéline est la démyélinisation.

17. Procédé selon la revendication 13, dans lequel le sujet ne reçoit pas de traitement pour le problème marqué par une altération de la régénération de la myéline avant la détermination de l'activité ou des niveaux d'IL4-i1.

18. Procédé selon l'une quelconque des revendications 13-17, dans lequel l'activité ou les niveaux d'IL4-i1 sont déterminés dans des macrophages activés.
